(19) Europäisches Patentamt
European Patent Office
Office européen des brevets

(11) EP 4 201 952 A1

(12) EUROPEAN PATENT APPLICATION

(43) Date of publication:
**28.06.2023 Bulletin 2023/26**

(21) Application number: **21383182.9**

(22) Date of filing: **21.12.2021**

(51) International Patent Classification (IPC):
***C07K 7/64*** *(2006.01)*

(52) Cooperative Patent Classification (CPC):
**C07K 7/645**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **CURIA SPAIN, S.A.U.**
**47151 Boecillo, Valladolid (ES)**

(72) Inventors:
- **CORDOVILLA LOSADA, Carlos**
  **47151 Boecillo, Valladolid (ES)**
- **FERNÁNDEZ SAINZ, María Yolanda**
  **47151 Boecillo, Valladolid (ES)**
- **LORENTE BONDE-LARSEN, Antonio**
  **47151 Boecillo, Valladolid (ES)**

(74) Representative: **ABG Intellectual Property Law, S.L.**
**Avenida de Burgos, 16D**
**Edificio Euromor**
**28036 Madrid (ES)**

# (54) PROCESS FOR THE CONTROLLED SYNTHESIS OF VOCLOSPORIN

(57) The invention relates to a process for the preparation of mixtures of (E) and (Z)-isomers of Voclosporin in a controlled manner.

EP 4 201 952 A1

## Description

## FIELD OF THE INVENTION

The invention relates to a process to obtain mixtures of (E) and (Z)-isomers of voclosporin and derivatives thereof in a controlled manner. Pharmaceutical compositions of Voclosporin enriched with the (E) isomer possess reduced toxicity over the individual isomers. The invention describes a synthetic process to obtain a customizable mixture of the (E) and (Z)-isomers directly from a single chemical procedure.

## BACKGROUND OF THE INVENTION

Voclosporin (ISATX247) is an immunosuppressant drug being developed by Aurinia Pharmaceuticals as a potential treatment for lupus nephritis (LN) and uveitis. It is a synthetic derivative of Cyclosporin A (isolated in 1970) where an additional carbon connected by a double bond is introduced.

Voclosporin is a mixture of cis and trans isomers of cyclosporin diene analogue, which is chemically described as cyclo((E5Z)-(2S,3R,4R)-3-hydoxy-4-memyl-2-(methylamino)-6,8-nonadienoyl-L-2-aminobutyryl- N-methyl-glycyl-N-methyl-L-leucyl-L-valyl-N-methyl-L-leucyl-L-alanyl-D-alanyl-N-methyl-L-leucyl-N-methyl-L-leucyl-N-methyl-L-valyl}.

It is remarkable that a mixture of ISATX247 isomers exhibits a combination of enhanced potency and reduced toxicity over natural cyclosporins and presently known cyclosporin derivatives (Abel et al., "ISATX247: A Novel Calcineurin Inhibitor," J. Heart Lung Transplant, 20:161 (2001); Aspeslet et al., "ISATX247: A Novel Calcineurin Inhibitor," Transplantation Proceedings, 33:1048-1051 (2001); US6605593 and US6613739).

The pharmaceutical product containing Voclosporin is defined as an enriched trans/(E) mixture, but the compound cannot be in a pure trans form due to its toxicity, instead, the cis/(Z) isomer is needed at least in small percentage.

Commercial Voclosporin is a mixture of the E and Z isomers, with the E isomer being prevalent (90-95%).

Accordingly, there is a need in the art for methods of preparation of voclosporin and its analogs. Synthetic pathways are needed that produce mixtures of the isomers having a desired ratio of the two isomers, especially customizable mixtures of trans/cis isomers (for example 90/10 or 95/5 or 96/4).

WO1999/018120 describes the preparation of deuterated and undeuterated cyclosporine analogues which can be used as immunomodulating agents. The synthetic route starting from naturally occurring cyclosporine involves the steps of acetylation of the hydroxyl group at the amino acid residue 1, oxidation with $OsO_4$ and $NaIO_4$, reaction with deuterated phosphonium derivatives (Witting reagents) and alkaline hydrolysis/O-deprotection. The overall sequence leads to an unspecified cis:trans ratio.

WO2003/033526 is related to isomeric mixtures of cyclosporin analogues and describes synthetic pathways for producing isomers of cyclosporin A (CsA) analogs, where such pathways vary in the degree of stereoselectivity.

In this document, acetyl CsA aldehyde is a key intermediate through several routes and it is reacted with Wittig reagents, phosphonium ylides, boronate esters, or organo-titanium reagent as sources of the additional C3 fragment in Voclosporin. Workup or elimination/hydrolysis leads to variable E/Z ratios of an acetyl-1,3-diene advanced intermediate. Final deprotection of the protected alcohol group gives Voclosporin isomeric mixtures with an unchanged E/Z ratio. The disadvantage of this method is that a certain E/Z ratio is introduced at the initial step (reaction with an allyl source) and is maintained throughout the sequence with many ratio values that are undesirable.

WO2004/089960 discloses the synthesis of voclosporin using as starting materials the protected aldehyde intermediate disclosed in WO2003/033526 and allyl boronates. Subsequent Peterson elimination generates anti or syn intermediates depending on the elimination conditions (acidic or basic) and hydrolysis of the alcohol protecting group affords Voclosporin as pure isomer.

WO2006014872 discloses a four-step synthesis of Voclosporin wherein the diene moiety is introduced through silver-catalyzed reaction of the corresponding aldehyde with an organo-zirconium reagent and deprotection. However, ten equivalents of the organometallic reagent are needed and only a moderate 47% product yield is achieved. Voclosporin is obtained as trans-only isomer.

WO2007112345 describes a process to afford halogenated Voclosporin derivatives from the acetyl protected cyclosporine A or a cyclosporine diol. Olefin metathesis is performed with 2$^{nd}$ generation Grubb's catalyst or Hoveyda-Grubb's catalyst. This step is stereoselective and affords an $\alpha,\beta$-unsaturated aldehyde exclusively as trans geometric isomer. Treatment of the $\alpha,\beta$-unsaturated aldehyde with haloform and CrCl2 in tetrahydrofuran provides a halogenated cyclosporin diene as a trans-isomer which is finally deprotected. Only a small amount of cis-isomer is observed under these conditions and additional steps are required to obtain Voclosporin.

Accordingly, there is a need in the art for methods to prepare trans-enriched isomeric mixtures of Voclosporin. In particular, synthetic pathways that produce the targeted ratio of the two isomers and allow for a better tuning of said ratio throughout the sequence are needed.

## SUMMARY OF THE INVENTION

The inventors observed that Wittig reaction of acetyl CsA aldehyde as disclosed in the prior art leads to (E) and (Z) mixtures of Voclosporin. Said reaction does not allow tuning said ratio as desired. On the other hand, reaction of acetyl CsA aldehyde with a boron reagent followed by Peterson elimination, as disclosed in WO2003/033526, gives rise to either the (E) or (Z) isomer. Therefore, subsequent mixture of the two mixtures is needed to obtain the desired (E) and (Z) mixtures of Voclosporin.

The inventors have found a process for the controlled preparation of (E) and (Z) mixtures of Voclosporin; in particular, (E) enriched mixtures.

This process comprises or consists of the following steps.

In a first step (step a)), an aldehyde of formula (II) is reacted with one equivalent or less of a compound of formula (III). The reaction between the compounds (II) and (III) leads to a mixture of (E) and (Z)-dienes (trans-(V) and cis-(V)). Due to the use of a low amount of compound of formula (III) in this step, part of the starting aldehyde of formula (II) remains unreacted.

The mixture obtained after this first step is then reacted with a silylallylborane reagent of formula (IV) and an acid (step b)). In this step, the unreacted aldehyde of formula (II) reacts with the borane reagent giving rise to a mixture of anti silylalcohols (VIa) and (VIb).

In the presence of an acid, elimination of the silylalcohols (VIa) and (VIb) occurs leading to the formation of additional (E)-diene (trans-(V)).

Cleavage of the protecting groups in the compounds of formula (V) (step c)) gives rise to the desired (E) and (Z) mixtures of Voclosporin isomers.

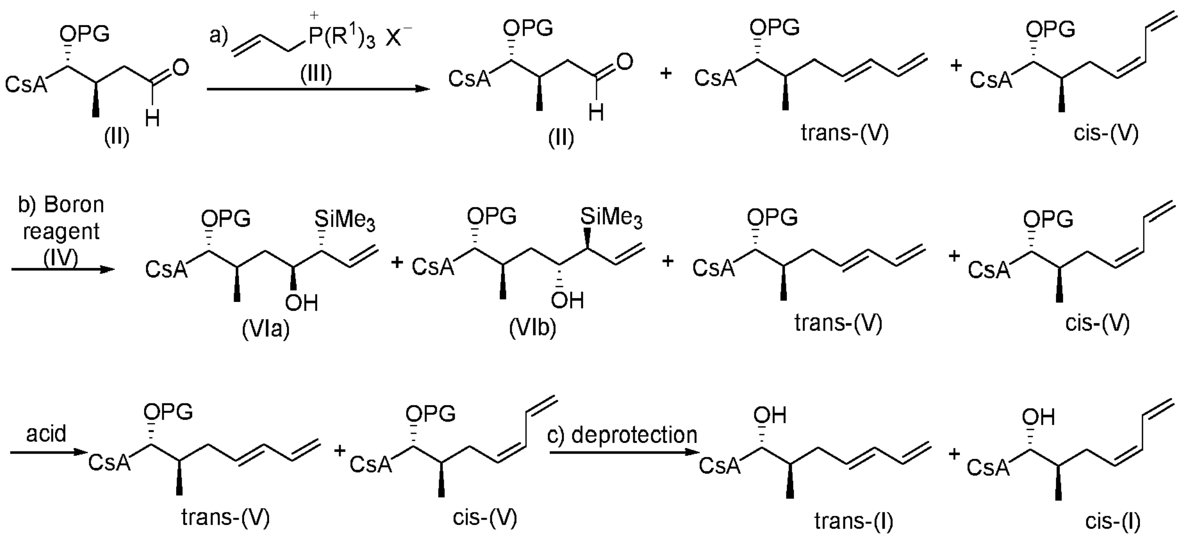


In step a) (Wittig reaction), a mixture of trans-(V) (mayor) and cis-(V) (minor) isomers is obtained, along with unreacted aldehyde (II). Through step b), the unreacted aldehyde is converted into the trans-(V) isomer via allylation and Peterson elimination. Therefore, the amount of cis-(V) isomer in the final mixture is determined by the amount of cis-(V) isomer formed in the Wittig reaction.

In this way, the inventors have found that the amount of cis-(V) isomer in the final mixture can be tuned by controlling the amount of compound of formula (III) in step a). Voclosporin with a higher ratio of (Z)/cis isomer can be obtained by increasing the amount of compound of formula (III), so that a lower amount of unreacted aldehyde is obtained (and subsequently converted into the (E)/trans isomer) after step a). On the contrary, Voclosporin with a lower ratio of (Z)/cis isomer can be obtained by decreasing the amount of compound of formula (III), so that a higher amount of unreacted aldehyde is obtained (and subsequently converted into the (E)/trans isomer) after step a).

Accordingly, the process of the invention allows to finely tune the (E):(Z) ratio in the final mixture.

Therefore, in a first aspect, the invention is directed to a process for preparing a mixture of (E) and (Z)-Voclosporin of formula (I)

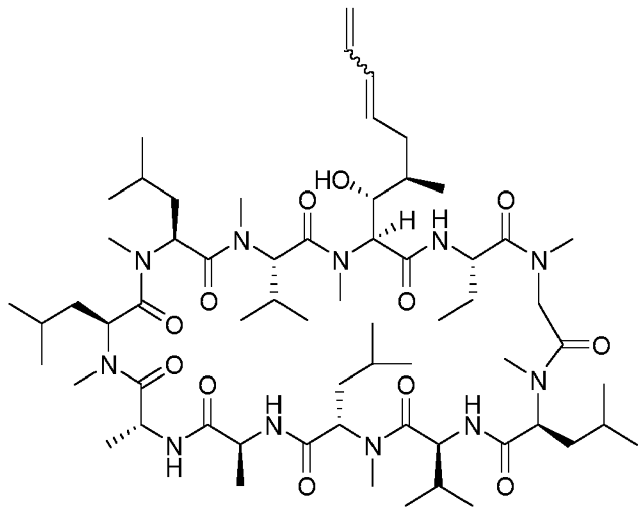

=

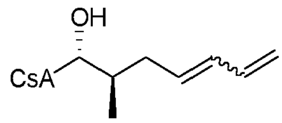

(I)

comprising:

a) reacting an aldehyde of formula (II),

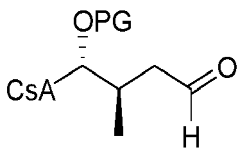

(II)

wherein PG is a hydroxyl protecting group and CsA represents the cyclosporine A ring as shown above, with a compound of formula (III)

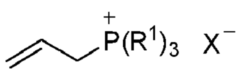

(III)

wherein

X is halogen, and
each $R^1$ is independently selected from $C_1$-$C_6$ alkyl and $C_6$-$C_{10}$ aryl,

in an amount of 0.2-1.0 molar equivalents with respect to the aldehyde of formula (II), in the presence of a base; and

b) reacting the product obtained from step a) with a compound of formula (IV)

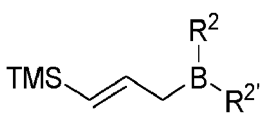

(IV)

wherein

$R^2$ and $R^{2'}$ are independently selected from the group consisting of $C_{1-6}$ alkyl, $C_{3-7}$ cycloalkyl, $OR^3$, or together they form a $C_{5-9}$ cycloalkyl group, or the -B($R^2R^{2'}$) group is -$BF_3K$; wherein each $R^3$ is independently selected from the group consisting of H, $C_{1-6}$ alkyl, $C_{3-7}$ cycloalkyl, or together the two $R^3$ groups form a group selected from $C_{3-7}$ cycloalkyl, 5- to 7-membered heterocycloalkyl, 5- to 7-membered heteroaryl, $C_{6-10}$ aryl and from $C_{2-5}$ alkylene optionally substituted by $C_{1-6}$ alkyl or -COO($C_{1-6}$ alkyl) wherein one of the carbon atoms in the $C_{2-5}$ alkylene group can be replaced by O, S or $NR^4$, wherein $R^4$ is selected from H and $C_{1-6}$ alkyl;
and an acid; and

c) deprotecting the hydroxyl protecting group.

## DETAILED DESCRIPTION OF THE INVENTION

Voclosporin has formula (I) as shown above. It is also described herein as:

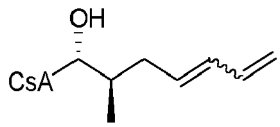

(I)

Therefore, the term "CsA" used herein represents the cyclosporine A ring, which corresponds to a moiety of formula:

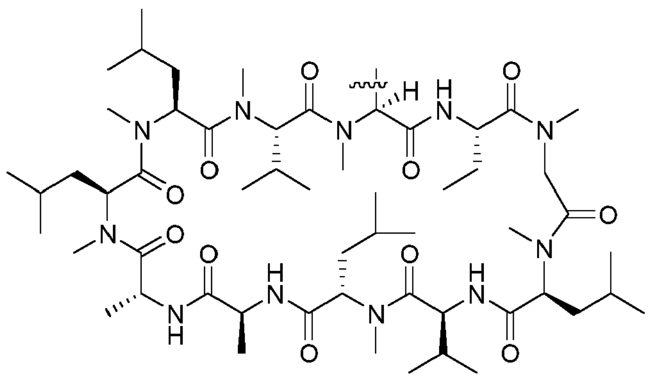

Throughout the text, "cis isomer" will be used as equivalent of "(Z)-isomer" and "trans isomer" will be used as equivalent of "(E)-isomer".

The percentage of one isomer or another in a mixture can be verified using nuclear magnetic resonance spectroscopy (e.g. 1H-NMR), high performance liquid chromatography (HPLC) or other techniques well known in the art. In a particular embodiment, the percentage of each isomer is determined by NMR, such as $^1H$-NMR.

The term "$C_1$-$C_6$ alkyl" refers to a linear or branched alkane derivative containing from 1 to 6, preferably from 1 to 3 ("$C_1$-$C_3$ alkyl"), carbon atoms and which is bound to the rest of the molecule through a single bond. Illustrative examples of alkyl groups include methyl, ethyl, n-propyl, i-propyl, n-butyl, i-butyl, t-butyl, pentyl, hexyl.

The term "cycloalkyl" refers to a radical derived from cycloalkane containing from 3 to 7 ("$C_3$-$C_7$ cycloalkyl"), preferably from 3 to 6 ("$C_3$-$C_6$ cycloalkyl"), or from 5 to 9 ("$C_5$-$C_9$ cycloalkyl"), preferably from 5 to 7 ("$C_5$-$C_7$ cycloalkyl"), carbon atoms. Illustrative examples of cycloalkyl groups include cyclopropyl, cyclobutyl, cyclopentyl, cyclohexyl, cycloheptyl, cyclooctyl, etc.

The term "$C_1$-$C_6$ alkoxyl" designates an alkyl group as defined above having between 1 and 6 carbon atoms, more preferably between 1 and 3 carbon atoms ("$C_1$-$C_3$ alkoxyl"), linked to the rest of the molecule through oxygen. Examples of alkoxy include methoxy, ethoxy, isopropoxy, tertbutoxy, and the like.

The term "$C_2$-$C_5$ alkylene" designates a divalent group having from two to five carbon atoms. These carbon atoms can be optionally substituted with one or more, preferably with one to four, $C_1$-$C_6$ alkyl or -COO($C_{1-6}$ alkyl) groups, and one of the carbon atoms in the alkylene group can be replaced by oxygen, sulfur or nitrogen. Examples of $C_2$-$C_5$ alkylene groups include $-CH_2-CH_2-$, $-CH_2-CH_2-CH_2-$, $-CH_2-CH_2-CH_2-CH_2-$, $-CH_2-CH_2-CH_2-CH_2-CH_2-$, -CH($COOC_{1-6}$ alkyl)-CH($COOC_{1-6}$ alkyl)-, $-CH(CH_3)-CH_2-$,$-CH(CH_3)-CH(CH_3)-$, $-C(CH_3)_2-CH(CH_3)-$, $-C(CH_3)_2-C(CH_3)_2-$, $-CH_2-C(CH_3)_2-CH_2-$, $-C(CH_3)_2-CH_2-C(CH_3)_2-$, $-CH_2-CH_2-O-CH_2-CH_2-$, $-CH_2-CH_2-S-CH_2-CH_2-$,$-CH_2-CH_2-NH-$

$CH_2$-$CH_2$-, -$CH_2$-$CH_2$- N($CH_3$)-$CH_2$-$CH_2$- $CH_2$-$CH_2$- N(tBu)-$CH_2$-$CH_2$-.

The term "$C_6$-$C_{10}$ aryl" refers to an aromatic group having between 6 and 10, preferably 6 or 10 carbon atoms, comprising 1 or 2 aromatic nuclei fused to one another. Illustrative examples of aryl groups include phenyl, naphthyl, indenyl, phenanthryl, etc. Preferably, it is phenyl.

The term "halogen" refers to bromine, chlorine, iodine or fluorine.

The term "heterocyclyl" refers to a saturated or partially unsaturated monocyclic or bicyclic system containing from 5 to 7 ring atoms containing one or more, specifically one, two or three ring heteroatoms independently selected from N, O, and S, and the remaining ring atoms being carbon.

The term "heteroaryl" refers to an aromatic monocyclic or bicyclic system containing from 5 to 7 ring atoms containing one or more, specifically one, two or three ring heteroatoms independently selected from O, N and S, and the remaining ring atoms being carbon.

The term "hydroxyl protecting group" refers to a group blocking the OH function for subsequent reactions that can be removed under controlled conditions. Hydroxyl protecting groups are well known in the art. Illustrative examples of hydroxyl protecting groups have been described by Green TW et al. in "Protective Groups in Organic Synthesis", 3rd Edition (1999), Ed. John Wiley & Sons. Virtually any hydroxyl protecting group can be used to put the invention into practice. Illustrative, non-limiting examples of hydroxyl protecting groups include:

- silyl ethers [-Si(R)(R')(R")]. R, R' and R" can be independently selected from $C_1$-$C_6$ alkyl, $C_3$-$C_7$ cycloalkyl, $C_6$-$C_{10}$ aryl, $C_1$-$C_6$ alkoxyl and halogen. Examples of silyl ethers include trimethylsilyl ether, triethylsilyl ether, tert-butyldimethylsilyl ether, tert-butyldiphenylsilyl ether, tri-isopropylsilyl ether, diethylisopropylsilyl ether, hexyldimethylsilyl ether, triphenylsilyl ether, di-tert-butylmethylsilyl ether;
- ethers [-R], including alkoxy and aryloxy methyl ethers [-$CH_2$-OR]. R can be selected from $C_1$-$C_6$ alkyl, $C_6$-$C_{10}$ aryl and ($C_6$-$C_{10}$)aryl($C_1$-$C_6$)alkyl. Examples of ethers include methyl ether, tert-butyl ether, benzyl ether, p-methoxybenzyl ether, 3,4-dimethoxybenzyl ether, trityl ether, allyl ether, methoxymethyl ether, 2-methoxyethoxymethyl ether, benzyloxymethyl ether, p-methoxybenzyloxymethyl ether, 2-(trimethylsilyl)ethoxymethyl ether; tetrahydropyranyl and related ethers;
- esters [-COR]. R can be selected from $C_1$-$C_6$ alkyl, $C_6$-$C_{10}$ aryl and ($C_6$-$C_{10}$)aryl($C_1$-$C_6$)alkyl. Examples of esters include acetate ester, propionate ester, butyrate ester, isobutyrate ester, valerate ester, benzoate ester, pivalate ester, methoxyacetate ester, chloroacetate ester, levulinate ester; and
- carbonates [-COOR]. R can be selected from $C_1$-$C_6$ alkyl, $C_6$-$C_{10}$ aryl and ($C_6$-$C_{10}$)aryl($C_1$-$C_6$)alkyl. Examples of carbonates include benzyl carbonate, p-nitrobenzyl carbonate, tert-butyl carbonate, 2,2,2-trichloroethyl carbonate, 2-(trimethylsilyl)ethyl carbonate, allyl carbonate.

As understood in this technical area, there may be a certain degree of substitution in the aforementioned radicals. Therefore, there may be substitution in any of the groups of the present invention. The previous groups can be substituted in one or more available positions with one or more substituents. Said substituents include, for example and in non-limiting sense, $C_{1-6}$ alkyl, $C_{3-7}$ cycloalkyl, $C_6$-$C_{10}$ aryl, 5- to 7-membered heterocyclyl, 5- to 7-membered heteroaryl, halogen, -CN, $NO_2$, $CF_3$, -N($R_a$)($R_b$), -O$R_c$, -S$R_d$, -C(O)$R_e$, -C(O)O$R_f$, -C(O)N($R_g$)($R_h$), -OC(O)$R_i$; wherein $R_a$, $R_b$, $R_c$, $R_d$, $R_e$, $R_f$, $R_g$, $R_h$ and $R_i$ are independently selected from hydrogen, $C_1$-$C_6$ alkyl, $C_6$-$C_{10}$ aryl, 5- to 7-membered heterocyclyl, 5- to 7-membered heteroaryl and trifluoromethyl.

The term "organic solvent" includes for example cyclic and acyclic ethers (e.g. $Et_2O$, $iPr_2O$, $tBu_2O$, MeOtBu, 1,4-dioxane, 1,3-dioxolane, 1,2-dimethoxyethane (DME), tetrahydrofuran (THF), methyltetrahydrofuran (MeTHF)), hydrocarbon solvents (e.g. pentane, hexane, heptane), halogenated solvents (e.g. dichloromethane, dichloroethane, chloroform), aromatic solvents (e.g. benzene, toluene, xylene), esters (e.g. EtOAc, nBuOAc, iPrOAc), ketones (e.g. acetone, methylethyl ketone, cyclohexanone), nitriles (e.g. acetonitrile), amides (e.g. DMF, DMA, NMP), alcohols (e.g. methanol, ethanol, n-propanol, i-propanol, t-butanol), sulfoxides (DMSO) and mixtures thereof.

In an aspect, the invention is directed to a process for preparing a mixture of (E) and (Z)-Voclosporin comprising:

a) reacting an aldehyde of formula (II),

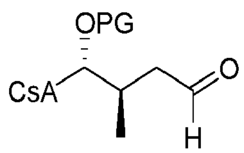

(II)

wherein PG is a hydroxyl protecting group,
with 0.2-1.0 molar equivalents of a compound of formula (III)

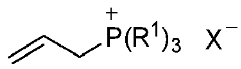

(III)

wherein

X is halogen, and
each $R^1$ is independently selected from $C_1$-$C_6$ alkyl and $C_6$-$C_{10}$ aryl,

with respect to the aldehyde of formula (II),
in the presence of a base; and

b) reacting the product obtained from step a) with a compound of formula (IV)

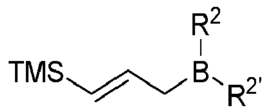

(IV)

wherein

$R^2$ and $R^{2'}$ are independently selected from the group consisting of $C_{1-6}$ alkyl, $C_{3-7}$ cycloalkyl, $OR^3$, or together they form a $C_{5-9}$ cycloalkyl group, or the -$B(R^2R^{2'})$ group is -$BF_3K$; wherein each $R^3$ is independently selected from the group consisting of H, $C_{1-6}$ alkyl, $C_{3-7}$ cycloalkyl, or together the two $R^3$ groups form a group selected from $C_{3-7}$ cycloalkyl, 5- to 7-membered heterocycloalkyl, 5- to 7-membered heteroaryl, $C_{6-10}$ aryl, and from $C_{2-5}$ alkylene optionally substituted by $C_{1-6}$ alkyl or -COO($C_{1-6}$ alkyl) wherein one of the carbon atoms in the $C_{2-5}$ alkylene group can be replaced by O, S or $NR^4$, wherein $R^4$ is selected from H and $C_{1-6}$ alkyl;
and an acid; and

c) deprotecting the hydroxyl protecting group.

In a preferred embodiment, the process of the invention gives rise to a mixture of isomers (E):(Z) in the ratio 87:13 to 97:3. More preferably, a mixture (E):(Z) from 90:10 to 97:3 or from 90:10 to 96:4 is obtained. In a more preferred embodiment, a mixture (E):(Z) from 90:10 to 95:5 is obtained.
In another embodiment, a mixture (E):(Z) from 87:13 to 94:6, preferably from 90:10 to 94:6, is obtained
As explained herein, the desired ratio of (E):(Z) isomers can be obtained by controlling the amount of Wittig reagent (compound of formula (III)) used in step a).
According to an embodiment of the invention, the hydroxyl protecting group (PG) is selected from:

- Si(R)(R')(R") where R, R' and R" are independently selected from $C_1$-$C_6$ alkyl, C3-C7 cycloalkyl, $C_6$-$C_{10}$ aryl, $C_1$-$C_6$ alkoxy and halogen,
- R or -$CH_2$-OR where R is selected from $C_1$-$C_6$ alkyl, $C_6$-$C_{10}$ aryl and ($C_6$-$C_{10}$)aryl($C_1$-$C_6$)alkyl,
- COR where R is selected from $C_1$-$C_6$ alkyl, $C_6$-$C_{10}$ aryl and ($C_6$-$C_{10}$)aryl($C_1$-$C_6$)alkyl, and
- COOR where R is selected from $C_1$-$C_6$ alkyl, $C_6$-$C_{10}$ aryl and ($C_6$-$C_{10}$)aryl($C_1$-$C_6$)alkyl;

In an embodiment, PG is selected from:

- Si(R)(R')(R") where R, R' and R" are independently selected from $C_1$-$C_6$ alkyl, $C_3$-$C_7$ cycloalkyl, $C_6$-$C_{10}$ aryl, $C_1$-$C_6$ alkoxy and halogen; such as trimethylsilyl (TMS), triethylsilyl (TES), tert-butyldimethylsilyl (TBDMS), tert-butyldiphenylsilyl (TBDPS), tri-isopropylsilyl (TIPS), diethylisopropylsilyl (DEIPS), hexyldimethylsilyl (THDMS), triphenylsilyl (TPS), di-tert-butylmethylsilyl (DTBMS); and

- COR where R is selected from $C_1$-$C_6$ alkyl, $C_6$-$C_{10}$ aryl and ($C_6$-$C_{10}$)aryl($C_1$-$C_6$)alkyl; such as acetyl, propionyl, butyryl, isobutyryl, valeryl, benzoyl, pivaloyl, methoxyacetyl, chloroacetatyl, levulinyl.

According to an embodiment, PG is a group of formula -COR where R is $C_1$-$C_6$ alkyl; such as $-COCH_3$ (Ac).
In an embodiment, PG is selected from Ac and TMS.

*Wittig reaction*

In step a) a Wittig reaction is conducted between the aldehyde of formula (II) and the compound of formula (III). In this step, a mixture of compound of formula (II), compound of formula trans-(V) and compound of formula cis-(V) is obtained as product.
X in the compound of formula (III) can be selected from Cl, Br and I.
Each $R^1$ in the compound of formula (III) is independently selected from $C_1$-$C_6$ alkyl and $C_6$-$C_{10}$ aryl; such as Me, Et, nPr, iPr, nBu, sBu, tBu or Ph.
In an embodiment, $R^1$ is selected from Ph and nBu.
In a further embodiment, the compound of formula (III) is selected from allyl tributylphosphonium bromide, allyl triphenylphosphonium bromide and allyl triphenylphosphonium iodide. In a particular embodiment, it is allyl tributylphosphonium bromide.
The base used in step a) can be selected from organolithium bases, organosodium bases, alkali metal hydrides, alkali metal hydroxides, alkali metal alkoxides, alkali metal carbonates and alkali metal phosphates, such as e.g. nBuLi, tBuLi, sBuLi, MeLi, HexLi, PhLi, HMDSLi, HMDSNa, LDA, NaH, LiOH, NaOH, KOH, LiOtBu, NaOtBu, KOtBu, LiOMe, LiOEt, NaOMe, NaOEt, KOMe, KOEt, $Na_2CO_3$, $K_2CO_3$, $Cs_2CO_3$, $Na_3PO_4$, $K_3PO_4$, and mixtures thereof.
In an embodiment, the base is selected from alkali metal hydroxides, alkali metal alkoxides and alkali metal carbonates, such as e.g. LiOH, NaOH, KOH, LiOtBu, NaOtBu, KOtBu, LiOMe, LiOEt, NaOMe, NaOEt, KOMe, KOEt, $Na_2CO_3$, $K_2CO_3$, $Cs_2CO_3$, and mixtures thereof. In a preferred embodiment, the base is selected from an alkali metal alkoxide, preferably it is KOtBu.
Step a) can be performed in the presence of an organic solvent.
In an embodiment, step a) is performed in the presence of an organic solvent selected from cyclic or acyclic ethers (e.g. $Et_2O$, $iPr_2O$, $tBu_2O$, MeOtBu, 1,4-dioxane, 1,3-dioxolane, 1,2-dimethoxyethane, tetrahydrofuran, methyltetrahydrofuran), hydrocarbon solvents (e.g. pentane, hexane, heptane), halogenated solvents (e.g. dichloromethane, dichloroethane, chloroform), aromatic solvents (e.g. toluene, xylene), ketones (e.g. acetone, methylethyl ketone, cyclohexanone), nitriles (e.g. acetonitrile), amides (e.g. DMF, DMA, NMP), sulfoxides (DMSO), and mixtures thereof.
In a particular embodiment, step a) is performed in the presence of a cyclic or acyclic ether as solvent, such as e.g. $Et_2O$, $iPr_2O$, $tBu_2O$, MeOtBu, 1,4-dioxane, 1,3-dioxolane, 1,2-dimethoxyethane, tetrahydrofuran, methyltetrahydrofuran; Toluene, Dichloromethane, acetonitrile, and mixtures thereof.
In an embodiment, the solvent in step a) is selected from cyclic or acyclic ethers (e.g. $Et_2O$, $iPr_2O$, $tBu_2O$, MeOtBu, 1,4-dioxane, 1,3-dioxolane, 1,2-dimethoxyethane, tetrahydrofuran, methyltetrahydrofuran) and halogenated solvents (e.g. dichloromethane, dichloroethane, chloroform). In an embodiment, the solvent is a cyclic ether, such as tetrahydrofuran (THF) or methyltetrahydrofuran (MeTHF).
The compound of formula (III) is used in the Wittig reaction in an amount of 0.2 to 1.0 molar equivalents with respect to the compound of formula (II). In this way, only part of the aldehyde of formula (II) reacts with the compound of formula (III) leading to a mixture of (E) and (Z) dienes. The unreacted aldehyde of formula (II) is subsequently transformed into the (E)-diene through step b).
In an embodiment, the compound of formula (III) is used in step a) in an amount of 0.3-1.0 molar equivalents with respect to the aldehyde of formula (II). That is, for each mol of compound of formula (II), 0.3-1.0 moles of compound of formula (III) are used in step a).
In a particular embodiment, the compound of formula (III) is used in step a) in an amount of 0.2-0.9 molar equivalents with respect to the aldehyde of formula (II). In a further embodiment, the compound of formula (III) is used in step a) in an amount of 0.2-0.8, or even 0.3-0.7, molar equivalents with respect to the aldehyde of formula (II).
The base is typically used in approximately the same molar amount as the compound of formula (III). In a particular embodiment, the molar amount of base is 90-95% de molar amount of compound of formula (III). Therefore, in an embodiment, the base in step a) is used in an amount of 0.18-0.95 molar equivalents with respect to the aldehyde of formula (II).
In another embodiment, the base in step a) is used in an amount of 0.27-0.95 molar equivalents with respect to the aldehyde of formula (II). In a further embodiment, the base in step a) is used in an amount of 0.18-0.76, or even 0.27-0.67, molar equivalents with respect to the aldehyde of formula (II).
In an embodiment, the reaction is performed at a temperature between -80°C and 60°C; preferably, between -50°C and 20°C; even more preferably, between -20°C and 20°C. In a preferred embodiment, the reaction is performed

at a temperature between - 20°C and 0°C.

According to an embodiment of the invention, the compound of formula (III) is allyl tributylphosphonium bromide and the base is KOtBu.

In a further embodiment, the compound of formula (III) is allyl tributylphosphonium bromide, the base is KOtBu, and the reaction is performed in the presence of an organic solvent, preferably a cyclic or acyclic ether.

In a further embodiment, the compound of formula (III) is allyl tributylphosphonium bromide, the base is KOtBu, and the reaction is performed in the presence of an organic solvent, preferably a cyclic or acyclic ether, at a temperature between -20°C and 20°C.

In another embodiment, the compound of formula (III) is allyl tributylphosphonium bromide, the base is KOtBu, and the reaction is performed in the presence of THF or MeTHF.

In a further embodiment, the compound of formula (III) is allyl tributylphosphonium bromide, the base is KOtBu, and the reaction is performed in the presence of THF or MeTHF at a temperature between -20°C and 20°C.

Aftertreatment of the reaction mixture obtained after the Wittig reaction can be performed as known in the art, for example by acid or basic treatment. In a particular embodiment, the reaction mixture obtained after the Wittig reaction is treated with an acid. Suitable acids include organic acids, inorganic acids and Lewis acids, such as phosphoric acid, sulfuric acid, methanesulfonic acid, acetic acid, trifluroacetic acid, formic acid, hydrochloric acid, trichloroacetic acid, tetrafluoroboric acid, chloric acid, perchloric acid, nitric acid, hydrobromic acid, $BF_3 \cdot THF$, $BF_3 \cdot OEt_2$, and mixtures thereof. This treatment can be performed at a temperature between 0°C and 30°C, for example between 10°C and 30°C.

*Reaction with borane reagent and Peterson elimination*

In step b) of the process of the invention, the product obtained after step a) is reacted with a compound of formula (IV)

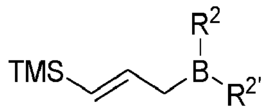

(IV)

wherein

$R^2$ and $R^{2'}$ are independently selected from the group consisting of:

- $C_{1-6}$ alkyl,
- $C_{3-7}$ cycloalkyl,
- $OR^3$, wherein each $R^3$ is independently selected from the group consisting of:
    - H,
    - $C_{1-6}$ alkyl,
    - $C_{3-7}$ cycloalkyl, or
    - together the two $R^3$ groups form a group selected from:
        - $C_{3-7}$ cycloalkyl,
        - 5- to 7-membered heterocycloalkyl,
        - 5- to 7-membered heteroaryl,
        - $C_{6-10}$ aryl, and
        - $C_{2-5}$ alkylene optionally substituted by $C_{1-6}$ alkyl or -COO($C_{1-6}$ alkyl) wherein one of the carbon atoms in the $C_{2-5}$ alkylene group can be replaced by O, S or $NR^4$, wherein $R^4$ is selected from H and $C_{1-6}$ alkyl, or
- together they form a $C_{5-9}$ cycloalkyl group, or
- the -B($R^2R^{2'}$) group is $-BF_3K$;

and with an acid.

The acid mentioned in step b) is used to promote anti-elimination of the silylalcohols (VIa) and (VIb) formed by

reaction of the product obtained in step a) with the compound of formula (IV).

After elimination, a mixture of compound of formula trans-(V) and compound of formula cis-(V) is obtained as product.

In a particular embodiment, this acid is an acid with a pKa lower than 4.

The term pKa refers to the negative logarithm (p) of the acid dissociation constant (Ka) of an acid, and is equal to the pH value at which equal concentrations of the acid and its conjugate base form are present in solution. As used herein, the term pKa refers to the pKa value in water as determined at room temperature and atmospheric pressure.

In an embodiment, the acid in step b) is selected from sulfuric acid, phosphoric acid, hydrochloric acid, formic acid, methanesulfonic acid, trifluoroacetic acid, trichloroacetic acid, tetrafluoroboric acid, chloric acid, perchloric acid, nitric acid, hydrobromic acid and mixtures thereof.

In another embodiment, the acid in step b) is selected from sulfuric acid, phosphoric acid, hydrochloric acid, formic acid, methanesulfonic acid, and mixtures thereof.

In an embodiment, the acid in step b) is sulfuric acid.

In an embodiment, the acid in step b) is used in an amount of 1.5-10 molar equivalents with respect to the amount of aldehyde of formula (II) used in step a). That is, for each mol of compound of formula (II) used in step a), 1.5-10 moles of the acid in step b) are used. In a particular embodiment, the acid in step b) is used in an amount of 1.5-7.0 equivalents with respect to the aldehyde of formula (II) used in step a).

The acid in step b) can be added at any stage of step b). For example, it can be added to the product obtained after step a) before it is reacted with the compound of formula (IV), along with the compound of formula (IV), or after reaction of the product obtained in step a) with the compound of formula (IV).

In a particular embodiment, the acid in step is added after reaction of the product obtained in step a) with the compound of formula (IV). In that case, step b) comprises:

b') reacting the product obtained from step a) with a compound of formula (IV)

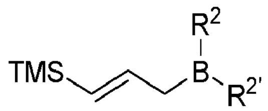

(IV)

and

b") treating the product obtained from step b') with an acid.

In an embodiment, the compound of formula (IV) is selected from:

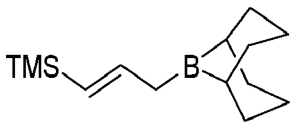
,
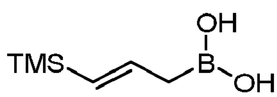
,
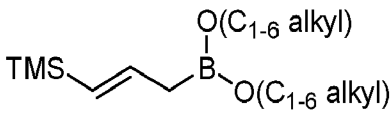
,
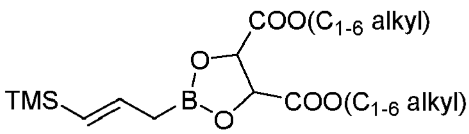
,
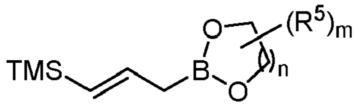
,
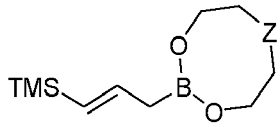

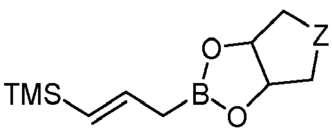
,
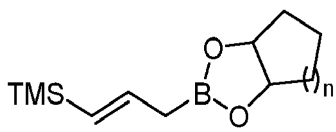
,
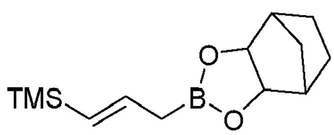

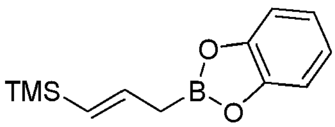 and 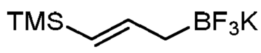

wherein

each $R^5$ is independently selected from $C_{1-6}$ alkyl,
n is an integer selected from 1 and 2,
m is an integer selected from 1, 2, 3 and 4, and
Z is selected from O, S and $NR^4$, wherein $R^4$ is selected from H and $C_{1-6}$ alkyl. In a particular embodiment, the compound of formula (IV) is selected from

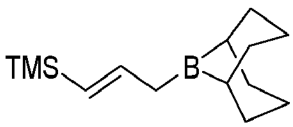, 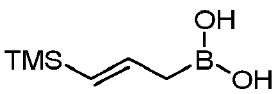, 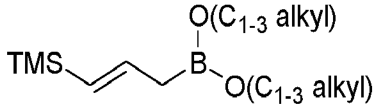,

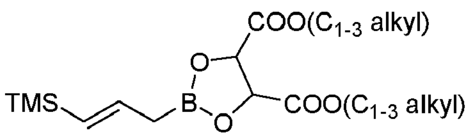, 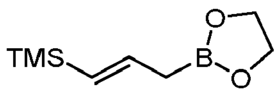, 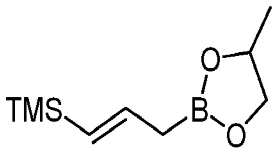

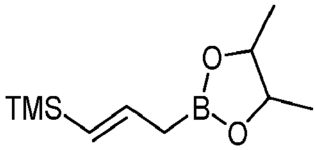, 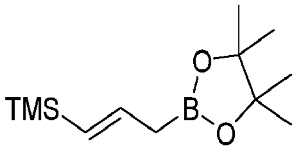, 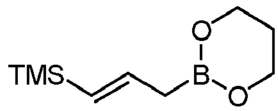

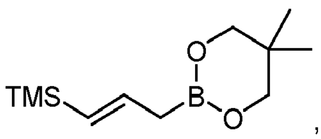, 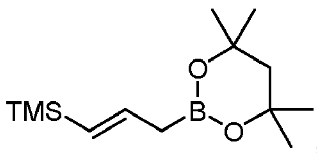, 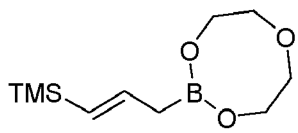

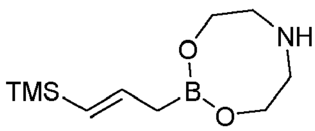, 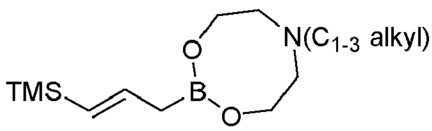,

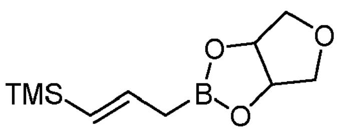

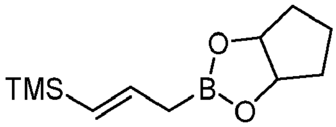, 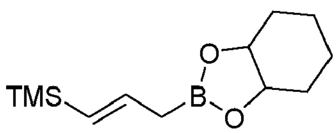, 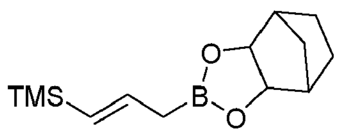

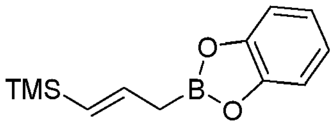 and 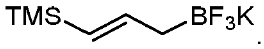

In an embodiment, the compound of formula (IV) is selected from

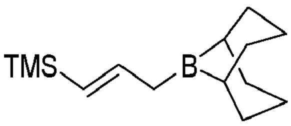, 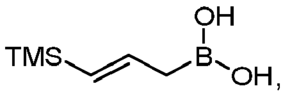, 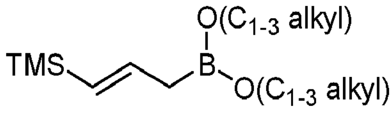,

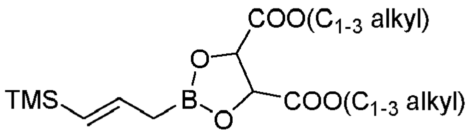, 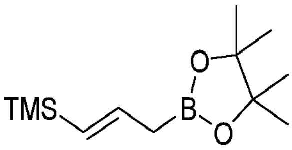, 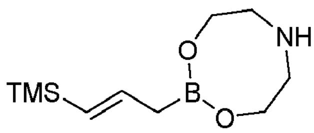

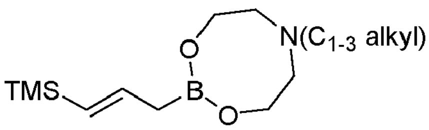, 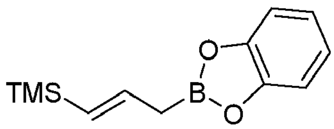

and

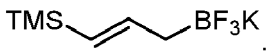.

In a further embodiment, the compound of formula (IV) is 4H-1,3,6,2-Dioxazaborocine, tetrahydro2-[(2E)-3-(trimethylsilyl)-2-propen-1-yl], with the following formula:

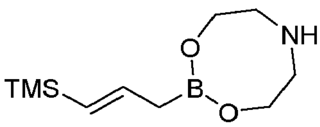.

In some embodiments, a further acid can be added in step b) to hydrolize the boron compound and liberate the (reactive) boronic acid, for example when a boronic ester is used as the compound of formula (IV).

Suitable acids for this purpose are well known by the skilled person and include organic acids, inorganic acids and Lewis acids, such as for example phosphoric acid, sulfuric acid, methanesulfonic acid, acetic acid, trifluroacetic acid, formic acid, hydrochloric acid, formic acid, trichloroacetic acid, tetrafluoroboric acid, chloric acid, perchloric acid, nitric acid, hydrobromic acid $BF_3 \cdot THF$, $BF_3 \cdot OEt_2$, and mixtures thereof. In an embodiment, this acid is selected from phosphoric acid, sulfuric acid, methanesulfonic acid, acetic acid, trifluroacetic acid, formic acid, $BF_3 \cdot THF$, $BF_3 \cdot OEt_2$, and mixtures thereof. In another embodiment, it is acetic acid.

This further acid might be the same as or different from the acid used in step b) to promote elimination of the silylalcohols (VIa) and (VIb).

In a particular embodiment, the acid used in step b) to promote elimination of the silylalcohols (VIa) and (VIb) is the same as the acid used to hydrolize (activate) the compound of formula (IV). In this case, it is preferably an acid with a pKa lower than 4, such as sulfuric acid, phosphoric acid, hydrochloric acid, formic acid, methanesulfonic acid, trifluoroacetic acid, trichloroacetic acid, tetrafluoroboric acid, chloric acid, perchloric acid, nitric acid, hydrobromic acid or mixtures thereof.

In a particular embodiment, the acid used in step b) to promote elimination of the silylalcohols (VIa) and (VIb) is different from the acid used to hydrolize (activate) the compound of formula (IV).

In a particular embodiment, the acid used to hydrolize (activate) the compound of formula (IV) is acetic acid

and the acid used in step b) to promote elimination of the silylalcohols (VIa) and (VIb) is sulfuric acid.

In an embodiment, the acid that might be used to hydrolize (activate) the compound of formula (IV) is used in an amount of 1.5-10 molar equivalents with respect to the amount of aldehyde of formula (II) used in step a). In a particular embodiment, the acid in step b) is used in an amount of 1.5-7.0 equivalents with respect to the aldehyde of formula (II) used in step a).

Step b) can be performed in the presence of an organic solvent.

In an embodiment, step b) is performed in the presence of an organic solvent selected from cyclic or acyclic ethers (e.g. $Et_2O$, $iPr_2O$, $tBu_2O$, MeOtBu, 1,4-dioxane, 1,3-dioxolane, 1,2-dimethoxyethane, tetrahydrofuran, methyltetrahydrofuran), hydrocarbon solvents (e.g. pentane, hexane, heptane), halogenated solvents (e.g. dichloromethane, dichloroethane, chloroform), aromatic solvents (e.g. toluene, xylene), esters (e.g. EtOAc, BuOAc, iPrOAc), ketones (e.g. acetone, methylethyl ketone, cyclohexanone), nitriles (e.g. acetonitrile), amides (e.g. DMF, DMA, NMP), alcohols (e.g. methanol, ethanol, propanol, i-propanol, t-butanol), sulfoxides (DMSO) and mixtures thereof; optionally in combination with water.

In a particular embodiment, step b) is performed in the presence of an organic solvent selected from cyclic or acyclic ethers (e.g. $Et_2O$, $iPr_2O$, $tBu_2O$, MeOtBu, 1,4-dioxane, 1,3-dioxolane, 1,2-dimethoxyethane, tetrahydrofuran, methyltetrahydrofuran), halogenated solvents (e.g. dichloromethane, dichloroethane, chloroform), esters (e.g. EtOAc, BuOAc, iPrOAc), aromatic solvents (e.g. toluene, xylene) and mixtures thereof; optionally in combination with water.

In an embodiment, step b) is performed in the presence of a halogenated solvent, such as e.g. dichloromethane, dichloroethane, chloroform, and mixtures thereof. In an embodiment, the solvent is dichloromethane (DCM).

In an embodiment, step b) is performed in the presence of a halogenated solvent, such as e.g. dichloromethane, dichloroethane, chloroform or mixtures thereof, and water. In an embodiment, step b) is performed in the presence of dichloromethane and water.

In an embodiment, step b) is performed in the presence of an aromatic solvent, such as e.g. toluene, xylene and mixtures thereof. In an embodiment, the solvent is toluene.

In an embodiment, step b) is performed in the presence of an aromatic solvent, such as e.g. toluene, xylene and mixtures thereof, and water. In an embodiment, the solvent is toluene and water.

In an embodiment, step b) is performed in the presence of a cyclic or acyclic ether as solvent, such as e.g. $Et_2O$, $iPr_2O$, $tBu_2O$, MeOtBu, 1,4-dioxane, 1,3-dioxolane, 1,2-dimethoxyethane, tetrahydrofuran, methyltetrahydrofuran, and mixtures thereof. In an embodiment, the solvent is a cyclic ether, such as tetrahydrofuran (THF) or methyltetrahydrofuran (MeTHF).

In an embodiment, step b) is performed in the presence of an ester as solvent, such as EtOAc, BuOAc, iPrOAc, and mixtures thereof. In an embodiment, the solvent is iPrOAc.

When step b) is conducted in a solvent different from step a), the reaction mixture after step a) might be subjected to replacement of the solvent.

When step b) is conducted in the same solvent as step a), the reaction mixture after step a) as such can be reacted with the compound of formula (IV).

In a particular embodiment, when the acid used in step b) is added after reaction of the product obtained in step a) with the compound of formula (IV), the reaction of the product obtained from step a) with a compound of formula (IV) (step b') and the subsequent treatment with an acid (step b") can be performed in the presence of the same solvent or in the present of different solvents for each step. If the same solvent is used in both steps, the reaction mixture after step b') as such can be reacted with the acid. If a different solvent is used, then the reaction mixture obtained after step b') might be subjected to replacement of the solvent before step b").

In an embodiment, the solvent used in step b') is as defined above in relation to step b).

In an embodiment, the solvent used in step b") is organic solvent selected from cyclic or acyclic ethers (e.g. $Et_2O$, $iPr_2O$, $tBu_2O$, MeOtBu, 1,4-dioxane, 1,3-dioxolane, 1,2-dimethoxyethane, tetrahydrofuran, methyltetrahydrofuran), hydrocarbon solvents (e.g. pentane, hexane, heptane), halogenated solvents (e.g. dichloromethane, dichloroethane, chloroform), aromatic solvents (e.g. toluene, xylene), esters (e.g. EtOAc, BuOAc, iPrOAc), ketones (e.g. acetone, methylethyl ketone, cyclohexanone), nitriles (e.g. acetonitrile), amides (e.g. DMF, DMA, NMP), sulfoxides (DMSO) and mixtures thereof.

In a particular embodiment, step b") is performed in the presence of a cyclic or acyclic ether as solvent, such as e.g. $Et_2O$, $iPr_2O$, $tBu_2O$, MeOtBu, 1,4-dioxane, 1,3-dioxolane, 1,2-dimethoxyethane, tetrahydrofuran, methyltetrahydrofuran, and mixtures thereof. In an embodiment, the solvent is a cyclic ether, such as tetrahydrofuran (THF), methyltetrahydrofuran (MeTHF) or a mixture thereof.

In an embodiment, the compound of formula (IV) is used in step b) in an amount of 1.5-10 molar equivalents with respect to the amount of aldehyde of formula (II) used in step a). That is, for each mol of compound of formula (II) used in step a), 1.5-10 moles of compound of formula (IV) are used in step b). In a particular embodiment, the compound of formula (IV) is used in an amount of 1.5-5.0, or of 1.8-3.0, equivalents with respect to the aldehyde of formula (II) used in step a).

In an embodiment, the acid is used in step b) in an amount of 1.5-10 molar equivalents with respect to the amount of aldehyde of formula (II) used in step a). In a particular embodiment, the acid in step b) is used in an amount of 1.5-6.0, or of 2.0-5.0, equivalents with respect to the aldehyde of formula (II) used in step a).

In an embodiment, the reaction is performed at a temperature between -80°C and 60°C; preferably, between -60°C and 40°C; even more preferably, between -20°C and 40°C. In a particular embodiment, the reaction is performed at a temperature between 10°C and 40°C, preferably, between 20 and 30°C.

According to an embodiment of the invention, step b) is performed in the presence of an acid and a mixture of an organic solvent and water.

In a further embodiment, step b') is performed in the presence of acetic acid and a mixture of toluene and water.

In an embodiment, step b') is performed in the presence of an organic solvent and acetic or trifluoroacetic acid.

In a further embodiment, step b') is performed in the presence of a cyclic or acyclic ether as solvent, preferably THF, MeTHF or a mixture thereof, and acetic or trifluoroacetic acid.

In a further embodiment, step b') is performed in the presence of THF, MeTHF or a mixture thereof, and acetic acid.

In a further embodiment, step b') is performed in the presence of trifluoroacetic acid and THF, MeTHF or a mixture thereof.

In an embodiment, step b) or b') is performed in the presence of sulfuric acid and THF, MeTHF or a mixture thereof, preferably at a temperature between 10°C and 40°C.

After step b) a mixture of compound of formula trans-(V) and compound of formula cis-(V) is obtained as product.

According to a particular embodiment, steps a)-b) in the process of the invention are performed in a one-pot manner. That is, without isolating the intermediate products.

In an embodiment, steps a) to b) are sequentially carried out in the presence of an organic solvent selected from cyclic or acyclic ethers, preferably THF, MeTHF or a mixture thereof.

In an embodiment, steps a) to b) are sequentially carried out in a one-pot manner in the presence of an organic solvent selected from cyclic or acyclic ethers, preferably THF, MeTHF or a mixture thereof.

*Cleavage of the hydroxyl protecting group*

Conversion of the mixture of the compound of formula trans-(V) and cis-(V) into Voclosporin (step c)) can be performed by deprotection of the hydroxyl protecting group as disclosed in the prior art (e.g. WO 1999/18120, WO 2003/033527, WO 2004/089960, WO 2006/014872).

Additionally, deprotection of the hydroxyl group in the compounds of formula trans-(V) and cis-(V) can be performed by conventional methods known by those skilled in the art (e.g. Green TW et al. in "Protective Groups in Organic Synthesis", 3rd Edition (1999), Ed. John Wiley & Sons (ISBN 0-471-16019-9)).

For example, compounds of formula trans-(V) and cis-(V) wherein OPG represents an ester (PG=COR) or a carbonate (PG=COOR) can be easily deprotected by hydrolysis in basic or acid media according to well-established procedures of the state of the art.

Compounds of formula trans-(V) and cis-(V) wherein OPG represents a silyl ether (PG=Si(R)(R')(R") can be deprotected by the use of fluoride reagents such as fluoride salts or HF, acid media, oxidizing media, etc.

Compounds of formula trans-(V) and cis-(V) wherein OPG represents an ether (PG=R, $CH_2OR$) can be easily deprotected through hydrolysis in acid media (for example, for methyl ethers (PG=$CH_2OR$)), hydrogenation (for example, for benzyl ethers), oxidation (for example, for aryl ethers), etc.

In a particular embodiment, OPG is an ester group (PG=COR, where R is selected from $C_1$-$C_6$ alkyl, $C_6$-$C_{10}$ aryl and ($C_6$-$C_{10}$)aryl($C_1$-$C_6$)alkyl) and it is deprotected by hydrolysis in basic or acid media; preferably by basic hydrolysis.

In a particular embodiment, when PG is a group of formula -CO($C_1$-$C_6$ alkyl), such as acetyl, propionyl, butyryl, isobutyryl 0 valeryl, it is deprotected by treatment with a base. Suitable bases include alkali metal hydroxides, alkali metal alkoxides and alkali metal carbonates, such as e.g. NaOH, KOH, NaOMe, NaOEt, NaOtBu, KOMe, KOEt, KOtBu, $Na_2CO_3$, $K_2CO_3$, $Cs_2CO_3$; preferably $K_2CO_3$.

In an embodiment, deprotection of said group is performed in the presence of a base and an organic solvent, for example a $C_{1-6}$ alcohol, and water.

According to an embodiment, PG is Ac and deprotection is performed in the presence of $K_2CO_3$, a $C_{1-6}$ alcohol (preferably MeOH) and water. This deprotection can be performed at a temperature between 0°C and 60°C, preferably between 10°C and 30°C.

Cleavage of the hydroxyl protecting group does not affect the (E)/(Z) ratio. Therefore, the same (E)/(Z) ratio obtained after step b) is maintained after deprotection of the protecting group in step c).

*Synthesis of the compound of formula (II)*

Preparation of the starting aldehyde of formula (II) is well-known in the art (e.g. WO 1999/18120, WO 2003/033527, WO 2004/089960, WO 2006/014872).

For example, the compound of formula (II) can be obtained from cyclosporine A, by: i) protecting the hydroxyl group and ii) converting the resulting compound into the aldehyde of formula (II):

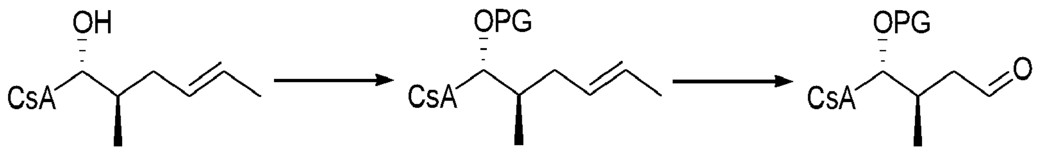

The protection of the hydroxyl group in step i) can be performed by conventional methods known by those skilled in the art (e.g. Green TW et al. in "Protective Groups in Organic Synthesis", 3rd Edition (1999), Ed. John Wiley & Sons (ISBN 0-471-16019-9)).

Step ii) can be performed in the presence of an oxidizing agent, such as ozone, periodic acid or periodate salt.

Alternatively, the compound of formula (II) can be obtained from cyclosporine A, by: i) protecting the hydroxyl group, ii) epoxidation of the double bond, and iii) oxidative cleavage of the epoxide to provide the aldehyde of formula (II):

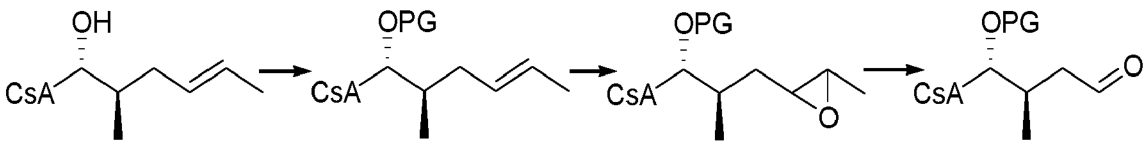

The protection of the hydroxyl group in step i) can be performed by conventional methods known by those skilled in the art (e.g. Green TW et al. in "Protective Groups in Organic Synthesis", 3rd Edition (1999), Ed. John Wiley & Sons (ISBN 0-471-16019-9)).

Step ii) can be performed in the presence of a peroxide, a peroxyacid or a persulfate.

Step iii) can be performed in the presence of an oxidizing agent, such as periodic acid or periodate salt under acidic conditions.

## EXAMPLES

The following examples are given to enable those skilled in the art to understand more clearly and to practice the present invention. They should not be considered as limiting the scope of the invention, but merely as being illustrative and representative thereof.

The cis and trans isomers were fully characterized by NMR ($^1H$, $^{13}C$, $^{13}C$_DEPT, COSY, NOESY; HSQC). Signals of $H^6$ and $H^7$ of MeBmt aminoacid are diagnostic for the determination of $C^6$-$C^7$ double bond configuration due to the differences in the coupling constant of $^3J(H^5-H^6)$ and $^3J(H^6-H^7)$ in voclosporin and in acetyl voclosporin:

cis: $H^6$ pseudotriplet: $^3J(H^5-H^6)_{cis} \sim {}^3J(H^6-H^7)$: 10 Hz;
trans: $H^6$ doblet of doblets $^3J(H^5-H^6)_{trans}$: 15 Hz and $^3J(H^6-H^7)$: 10 Hz;
and also due to the differences in the chemical shift of $H^7$ in the $^1H$-NMR:
acetyl voclosporin:

cis: $\delta$ $H^7$: 6.47 ppm;
trans: $\delta$ $H^7$: 6.27 ppm;

voclosporin:

cis: $\delta$ $H^7$: 6.55 ppm;
trans: $\delta$ $H^7$: 6.29 ppm.

The %cis isomer was calculated by 1H NMR by virtue of the values of the integration of the signals of H7 of both isomers according to the following formula:

$$\%cis = \frac{\mathrm{I(H7)}cis}{\mathrm{I(H7)}cis + \mathrm{I(H7)}trans} * 100$$

**Example 1.** Acetylation of Cyclosporine A

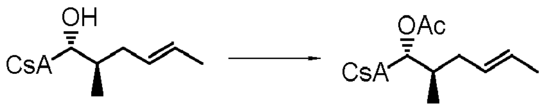

Cyclosporin A 1

Dichloromethane (200 g) was added to Cyclosporin A (30 g) and the mixture stirred at room temperature under a $N_2$ atmosphere until all of the starting material had dissolved. Acetic anhydride (12.73 g) and dimethylaminopyridine (4.57 g) were added and the reaction refluxed under a $N_2$ atmosphere until the reaction was complete (16-24 h). A solution of aqueous HCl (3.5 %) (300 mL) was added and stirred for 30 minutes, the organic phase was separated, treated with water (300 mL) and stirred for 30 minutes. The resulting organic phase was used for the next step without further purification.

**Example 2.** Epoxidation of compound 1

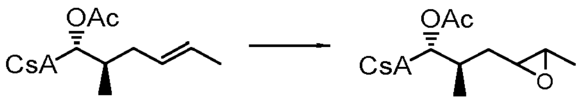

1 2

Hydrogen peroxide (30%) (12.17 g) and acetic anhydride (29.23 g) were added to the solution of compound **1** in dichloromethane obtained in the previous step and the reaction stirred at 30°C under a $N_2$ atmosphere until the reaction was complete (16 h). Water (110 mL) was added and stirred for 30 minutes, the organic phase was separated, treated with an aqueous solution of sodium sulfite (14%, 120 mL) and stirred for 30 minutes. The resulting organic phase was distilled until the designated volume is attained (83 mL). Acetone (118 g) was charged and the mixture was distilled to 83 mL. Acetone (118 g) was charged and the mixture was distilled to 83 mL. Acetone (118 g) was charged and the mixture was distilled to 3 83 mL. The resulting clear solution was stirred while water (150 mL) was added to give a suspension which was stirred for 30 minutes. The solid was collected by filtration and washed with acetone/water (3/5, 138 g). The solid product was dried in a vacuum oven overnight (30°C) to give product 2 as a colorless solid (95 %w/w. 28.2 g).

**Example 3.** Oxidation of compound **2**

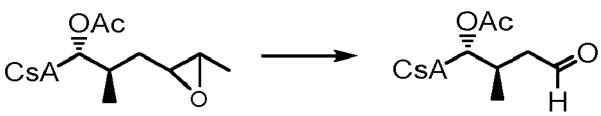

2 3

Acetonitrile (289 g) was added to compound **2** (28.2 g) and the mixture stirred at room temperature under a $N_2$ atmosphere until **2** was completely dissolved. Water (293 mL), concentrated sulfuric acid (2.2 g) and sodium periodate (7.18 g) were sequentially added. The mixture was stirred at 40°C under a $N_2$ atmosphere until the reaction was complete (3 h). Dichloromethane (188 g) was added and the mixture stirred for 30 minutes at room temperature. The resulting aqueous phase was discarded and the organic phase was distilled until the designated volume was attained (90 mL). Acetone (112 g) was charged and the mixture was distilled to 3 mL / g (82 mL). The same step was repeated three

times. Acetone (44.5 g) was charged and the resulting clear solution was stirred while water (197.5 mL) was added slowly at room temperature to give a suspension which was stirred for 18 hours. The solids were collected by filtration and washed with acetone/water (5/7) (128.6 g). The solid product was dried in a vacuum oven overnight (30°C) to give a white solid in approximately 60%w/w (16.9 g).

**Example 4:** Preparation of a **87:13** mixture of (E) and (Z) acetyl voclosporin. (Sequential Wittig, trans-boron compound addition, and Peterson elimination)

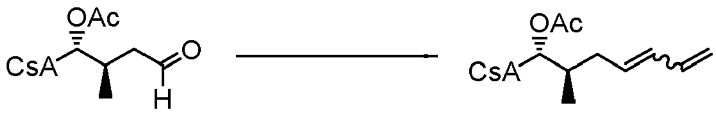

**3** **4**

Cyclosporine-aldehyde **3** (1.0 g) was added to tetrahydrofuran (10 mL) and the mixture stirred until the material was completely dissolved. To the resulting solution allyl tributylphosphonium bromide (260 mg, 1 equivalent) was added and the mixture was cooled at -10°C. Potassium tert-butoxide (82 mg, 0.92 equivalent) was added and the resulting mixture was stirred for 1 hour. After reaction completion, phosphoric acid (85%wt, 45 μL) was added and the solution warmed up to room temperature. A batch distillation for solvent exchange was performed to change tetrahydrofuran with dicloromethane (4 mL). Boron complex 4H-1,3,6,2-Dioxazaborocine, tetrahydro2-[(2E)-3-(trimethylsilyl)-2-propen-1-yl] was added (0.45 g, 2.03 equivalent). Then, water was added (2 mL) followed by the addition of acetic acid (0.14 mL, 3 equivalent). The reaction mixture was stirred 16 hours at 20/30 °C. After completion, the aqueous phase was discarded. A batch distillation for solvent exchange was performed to change dicloromethane with tetrahydrofuran (5 mL). Concentrated sulfuric acid (90 μL, 2 equivalent) was added and the mixture was stirred at 20/30 °C for 4 hours. After reaction completion, tert-butyl- methyl ether (10 mL) was added. After stirring (30 min) the aqueous phase was discarded. A batch distillation for solvent exchange was performed to change tert-butyl- methyl ether by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (87:13).

**Example 5:** Preparation of a **92:8** mixture of (E) and (Z) acetyl voclosporin. (Sequential Wittig, trans-boron compound addition, and Peterson elimination).

Cyclosporine-aldehyde 3 (1.0 g) was added to tetrahydrofuran (10 mL) and the mixture stirred until the material was completely dissolved. To the resulting solution allyl tributylphosphonium bromide (130 mg, 0.5 equivalent) was added and the mixture was cooled at -10°C. Potassium tertbutoxide (41 mg, 0.46 equivalent) was added and the resulting mixture was stirred for 1 hour. After reaction completion, phosphoric acid (85%, 45 μL) was added and warmed to room temperature. A batch distillation for solvent exchange was performed to change tetrahydrofuran by dicloromethane (4 mL). Boron complex (4H-1,3,6,2-Dioxazaborocine, tetrahydro2-[(2E)-3-(trimethylsilyl)-2-propen-1-yl]-) was added (0.45 g, 2.03 equivalent). Then, water was added (2 mL) followed by the addition of acetic acid (0.14 mL, 3 equivalent). The reaction mixture was stirred 16 hours at 20/30 °C. After completion, the aqueous phase was discarded. A batch distillation for solvent exchange was performed to change dicloromethane by tetrahydrofuran (5 mL). Concentrated sulfuric acid (90 μL, 2 equivalent) was added and the mixture was stirred at 20/30 °C for 4 hours. After reaction completion, tert-butyl- methyl ether (10 mL) was added. After stirring (30 min) the aqueous phase was discarded. A batch distillation for solvent exchange was performed to change tert-butyl- methyl ether by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (92:8).

**Example 6:** Preparation of a **93:7** mixture of (E) and (Z) acetyl voclosporin. (Sequential Wittig, trans-boron compound addition, and Peterson elimination).

Cyclosporine-aldehyde **3** (1.0 g) was added to tetrahydrofuran (10 mL) and the mixture stirred until the material was completely dissolved. To the resulting solution allyl tributylphosphonium bromide (100 mg, 0.4 equivalent) was added and the mixture was cooled at -10°C. Potassium tertbutoxide (34 mg, 0.37 equivalent) was added and the resulting mixture was stirred for 1 hour. After reaction completion, phosphoric acid (85%, 45 μL) was added and warmed until

room temperature. A batch distillation for solvent exchange was performed to change tetrahydrofuran by dicloromethane (4 mL). Boron complex (4H-1,3,6,2-Dioxazaborocine, tetrahydro2-[(2E)-3-(trimethylsilyl)-2-propen-1-yl]-) was added (0.45 g, 2.03 equivalent). Then, water was added (2 mL) followed by the addition of acetic acid (0.14 mL, 3 equivalent). The reaction mixture was stirred 16 hours at 20/30 °C. After completion, the aqueous phase was discarded. A batch distillation for solvent exchange was performed to change dicloromethane by tetrahydrofuran (5 mL). Concentrated sulfuric acid (90 μL, 2 equivalent) was added and the mixture was stirred at 20/30 °C for 4 hours. After reaction completion, tert-butyl- methyl ether (10 mL) was added. After stirring (30 min) the aqueous phase was discarded. A batch distillation for solvent exchange was performed to change tert-butyl- methyl ether by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (93:7)

**Example 7:** Preparation of a **94:6** mixture of (E) and (Z) acetyl voclosporin. (Sequential Wittig, trans-boron compound addition, and Peterson elimination).

Cyclosporine-aldehyde **3** (1.0 g) was added to tetrahydrofuran (10 mL) and the mixture stirred until the material was completely dissolved. To the resulting solution allyl tributylphosphonium bromide (78 mg, 0.3 equivalent) was added and the mixture was cooled at -10°C. Potassium tertbutoxide (26 mg, 0.28 equivalent) was added and the resulting mixture was stirred for 1 hour. After reaction completion, phosphoric acid (85%, 45 μL) is added and warmed to room temperature. A batch distillation for solvent exchange was performed to change tetrahydrofuran by dicloromethane (4 mL). Boron complex (4H-1,3,6,2-Dioxazaborocine, tetrahydro2-[(2E)-3-(trimethylsilyl)-2-propen-1-yl]-) was added (0.45 g, 2.03 equivalent). Then, water was added (2 mL) followed by the addition of acetic acid (0.14 mL, 3 equivalent). The reaction mixture was stirred 16 hours at 20/30 °C. After completion, the aqueous phase was discarded. A batch distillation for solvent exchange was performed to change dicloromethane by tetrahydrofuran (5 mL). Concentrated sulfuric acid (90 μL, 2 equivalent) was added and the mixture was stirred at 20/30 °C for 4 hours. After reaction completion, tert-butyl-methyl ether (10 mL) was added. After stirring (30 min) the aqueous phase was discarded. A batch distillation for solvent exchange was performed to change tert-butyl- methyl ether by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (94:6).

**Example 8:** Alternative preparation of a **92:8** mixture of (E) and (Z) acetyl voclosporin. (Sequential Wittig, trans-boron compound addition, and Peterson elimination).

Cyclosporine-aldehyde **3** (1.0 g) was added to tetrahydrofuran (5 mL) and the mixture stirred until the material was completely dissolved. To the resulting solution allyl tributylphosphonium bromide (130 mg, 0.5 equivalent) was added and the mixture was cooled at -10°C. Potassium tertbutoxide (42 mg, 0.46 equivalent) was added and the resulting mixture was stirred for 1 hour. After reaction completion, trifluoroacetic acid (0.37 mL, 6 equivalent) was added and warmed to room temperature. Boron complex (4H-1,3,6,2-Dioxazaborocine, tetrahydro2-[(2E)-3-(trimethylsilyl)-2-propen-1-yl]-) was added (0.45 g, 2.03 equivalent). After reaction completion (16 hours, room temperature), concentrated sulfuric acid (180 μL, 4 equivalent) was added and the mixture was stirred at room temperature for 16 hours. A batch distillation for solvent exchange was performed to change tetrahydrofuran by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (92:8).

**Example 9:** Alternative preparation of a **92:8** mixture of (E) and (Z) acetyl voclosporin. (Sequential Wittig, trans-boron compound addition, and Peterson elimination).

Cyclosporine-aldehyde **3** (1.0 g) was added to methyl-tetrahydrofuran (5 mL) and the mixture stirred until the material was completely dissolved. To the resulting solution allyl tributylphosphonium bromide (130 mg, 0.5 equivalent) was added and the mixture was cooled at -10°C. Potassium tertbutoxide (42 mg, 0.46 equivalent) was added and the resulting mixture was stirred for 1 hour. After reaction completion, trifluoroacetic acid (0.37 mL, 6 equivalent) was added and warmed to room temperature. Boron complex 4H-1,3,6,2-Dioxazaborocine, tetrahydro2-[(2E)-3-(trimethylsilyl)-2-propen-1-yl] was added (0.45 g, 2.03 equivalent). After reaction completion (16 hours, room temperature), water was added (10 mL) and the aqueous phase was discarded. Batch azeotropic distillation was performed to reach a content of water lower than 0,5%. Concentrated Sulfuric acid (90 μL, 2 equivalent) was added and the mixture was stirred for 4 hours. After reaction completion, a batch distillation for solvent exchange was performed to change methyl-tetrahydrofuran by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (92:8).

**Example 10:** Alternative preparation of a **92:8** mixture of (E) and (Z) acetyl voclosporin. (Sequential Wittig, trans-boron compound addition, and Peterson elimination).

Cyclosporine-aldehyde **3** (1.0 g) was added to methyl-tetrahydrofuran (5 mL) and the mixture stirred until the material was completely dissolved. To the resulting solution allyl tributylphosphonium bromide (130 mg, 0.5 equivalent) was added and the mixture was cooled at -10°C. Potassium tertbutoxide (42 mg, 0.46 equivalent) was added and the resulting mixture was stirred for 1 hour. After reaction completion, boron trifluoride tetrahydrofuran complex (0.18 mL, 2 equivalent) was added and warmed to room temperature. Boron complex 4H-1,3,6,2-Dioxazaborocine, tetrahydro2-[(2E)-3-(trimethylsilyl)-2-propen-1-yl] was added (0.45 g, 2.03 equivalent). After reaction completion (2 hours, room temperature), concentrated sulfuric acid (180 μL, 4 equivalent) was added and the mixture was stirred for 16 hours. After reaction completion, a batch distillation for solvent exchange was performed to change methyl-tetrahydrofuran by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (92:8).

**Example 11:** Alternative preparation of a **92:8** mixture of (E) and (Z) acetyl voclosporin. (Sequential Wittig, and one-pot trans-boron compound addition and Peterson elimination).

Cyclosporine-aldehyde 3 (1.0 g) was added to methyl-tetrahydrofuran (5 mL) and the mixture stirred until the material was completely dissolved. To the resulting solution allyl tributylphosphonium bromide (130 mg, 0.5 equivalent) was added and the mixture was cooled at -10°C. Potassium tertbutoxide (42 mg, 0.46 equivalent) was added and the resulting mixture was stirred for 1 hour. After reaction completion, concentrated sulfuric acid (0.13 mL, 3 equivalent) was added and warmed to room temperature. Boron complex 4H-1,3,6,2-Dioxazaborocine, tetrahydro2-[(2E)-3-(trimethylsilyl)-2-propen-1-yl] was added (0.45 g, 2.03 equivalent). After reaction completion (16 hours, room temperature), a batch distillation for solvent exchange was performed to change methyl-tetrahydrofuran by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (92:8).

**Example 12:** Alternative preparation of a **92:8** mixture of (E) and (Z) acetyl voclosporin. (Sequential Wittig, and one-pot trans-boron compound addition and Peterson elimination).

Cyclosporine-aldehyde **3** (1.0 g) was added to methyl-tetrahydrofuran (5 mL) and the mixture stirred until the material was completely dissolved. To the resulting solution allyl tributylphosphonium bromide (130 mg, 0.5 equivalent) was added and the mixture was cooled at -10°C. Potassium tertbutoxide (42 mg, 0.46 equivalent) was added and the resulting mixture was stirred for 1 hour. After reaction completion, methanosulfonic acid (0.32 mL, 6 equivalent) was added and warmed to room temperature. Boron complex 4H-1,3,6,2-Dioxazaborocine, tetrahydro2-[(2E)-3-(trimethylsilyl)-2-propen-1-yl] was added (0.45 g, 2.03 equivalent). After reaction completion (16 hours, room temperature), a batch distillation for solvent exchange was performed to change methyl-tetrahydrofuran by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (92:8).

**Example 13:** Alternative preparation of a **92:8** mixture of (E) and (Z) acetyl voclosporin. (Sequential Wittig, and one-pot trans-boron compound addition and Peterson elimination).

Cyclosporine-aldehyde **3** (1.0 g) was added to tetrahydrofuran (5 mL) and the mixture stirred until the material was completely dissolved. To the resulting solution allyl tributylphosphonium bromide (130 mg, 0.5 equivalent) was added and the mixture was cooled at -10°C. Potassium tertbutoxide (42 mg, 0.46 equivalent) was added and the resulting mixture was stirred for 1 hour. After reaction completion, phosphoric acid (85%wt, 45 μL) was added and warmed to room temperature. The organic phase was concentrated and the residue was dissolved in isopropyl acetate (2 mL) and acetic acid (1.25 mL) and formic acid (1.25 mL) were added. Boron complex 4H-1,3,6,2-Dioxazaborocine, tetrahydro2-[(2E)-3-(trimethylsilyl)-2-propen-1-yl] was added (0.45 g, 2.03 equivalent). After reaction completion (48

hours, room temperature), a batch distillation for solvent exchange was performed to change by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (92:8).

**Example 14:** Preparation of a **91:9** mixture of (E) and (Z) acetyl voclosporin. (Sequential Wittig, trans-boron compound addition, and Peterson elimination).

Cyclosporine-aldehyde **3** (1.0 g) was added to dichloromethane (10 mL) and the mixture stirred until the material was completely dissolved. To the resulting solution allyl tributylphosphonium bromide (156 mg, 0.6 equivalent) was added and the mixture was cooled at -10°C. Potassium tertbutoxide (51 mg, 0.56 equivalent) was added and the resulting mixture was stirred for 2 hours. After reaction completion, acetic acid (0.19 mL, 4 equivalent) and water (2 mL) were added and warmed to room temperature. Boron complex 4H-1,3,6,2-Dioxazaborocine, tetrahydro2-[(2E)-3-(trimethylsilyl)-2-propen-1-yl] was added (0.45 g, 2.03 equivalent). After reaction completion (16 hours, room temperature), aqueous phase was discarded and the reaction mass was cooled to 0 °C. Sulfuric acid was added (0.09 mL, 2 equivalent). After 4 hours, a batch distillation for solvent exchange was performed to change dichloromethane by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (91:9).

**Example 15.** Deprotection of compound **4**

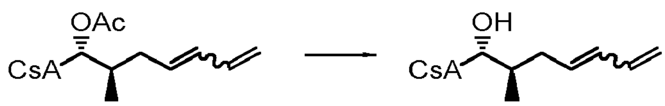

**4** **5**

Compound **4** (15.24 g, 12.12 mmol, ratio E/Z 92:8) was dissolved in methanol (305 mL) and then water (61 mL) was added. Potassium carbonate (16.76 g, 121.2 mmol) was added and the mixture stirred at room temperature for 16 hours until the reaction was complete. Formic acid (9.15 mL, 242.5 mmol) was added and the solvent was evaporated until a volume of 198 mL was reached (13 mL / g). Water (350 mL) was added and the resulting suspension was stirred for 2 hours. The supension was filtered and the solid was washed with 70 mL of a mixture of methanol/water (35/65). The resulting wet cake was dried in a vacuum oven overnight (50°C) to give crude Voclosporin as a mixture of isomers E:Z 92:8 (94% w/w, 14.32 g). The crude product was purified by column chromatography over silica gel using 40% acetone / 60% hexane as eluent to afford the final product with the same ratio of isomers.

**Example 16.** Deprotection of compound **4**

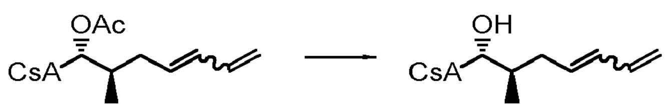

**4** **5**

Compound **4** (0.7 g, ratio E/Z 92:8) was dissolved in methanol (14 mL) and then water (2.8 mL) was added. Potassium hydroxide (0.31 g) was added and the mixture stirred at room temperature for 2 hours until the reaction was complete. Most of the methanol was evaporated and then ethyl acetate (14 mL) was added with stirring. A 10% aqueous citric acid solution (14 mL) was slowly added and then the ethyl acetate phase separated. The organic phase was washed sequentially with water (14 mL), 10% aqueous citric acid solution (14 mL) and saturated aqueous sodium chloride (14 mL). The ethyl acetate was evaporated to give a solid. Crude Voclosporin was isolated as a mixture of isomers E:Z 92:8.

**Example 17.** Deprotection of compound **4**

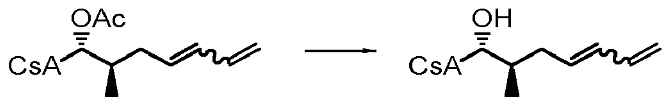

**4** **5**

Compound **4** (1 g, ratio E/Z 92:8) was dissolved in methanol (20 mL) and then water (4 mL) was added. Cesium carbonate (2.6 g) was added and the mixture stirred at room temperature for 24 hours until the reaction was complete. Most of the methanol was evaporated and then ethyl acetate (20 mL) was added with stirring. A 10% aqueous citric acid solution (20 mL) was slowly added and then the ethyl acetate phase separated. The organic phase was washed sequentially with water (20 mL), 10% aqueous citric acid solution (20 mL) and saturated aqueous sodium chloride (20 mL). The ethyl acetate was evaporated to give a solid. Crude Voclosporin was isolated as a mixture of isomers E:Z isomers E:Z 92:8.

**Comparative Example 18:** Preparation of (E) acetyl voclosporin (process similar to example 3 in WO 2004/089960)

Cyclosporine-aldehyde **3** (1.0 g) was added to dicloromethane (4 mL). Boron complex (4H-1,3,6,2-Dioxazaborocine, tetrahydro2-[(2E)-3-(trimethylsilyl)-2-propen-1-yl]-) was added (0.45 g, 2.03 equivalent). Then, water was added (2 mL) followed by the addition of acetic acid (0.14 mL, 3 equivalent). The reaction mixture was stirred 16 hours at 20/30 °C. After completion, the aqueous phase was discarded. A batch distillation for solvent exchange was performed to change dicloromethane by tetrahydrofuran (5 mL). Concentrated sulfuric acid (90 μL, 2 equivalent) was added and the mixture was stirred 4 hours. After reaction completion, tert-butyl-methyl ether (10 mL) was added: After stirring (30 min) the aqueous phase was discarded. A batch distillation for solvent exchange was performed to change tert-butyl- methyl ether by methanol (6 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (100:0).

**Comparative Example 19:** Preparation of a **82:18** mixture of (E) and (Z) acetyl voclosporin (Wittig reaction)

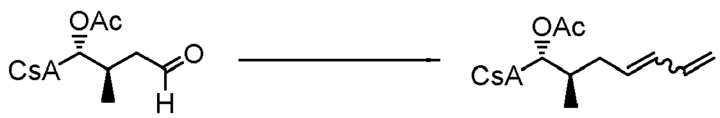

**3** **4**

Cyclosporine-aldehyde **3** (1.0 g) was added to tetrahydrofuran (10 mL) and the mixture stirred until the material was completely dissolved. To the resulting solution allyl tributylphosphonium bromide (680 mg, 2.6 equivalent) was added and the mixture was cooled at -10 °C. Potassium tert-butoxide (220 mg, 2.4 equivalent) was added and the resulting mixture was stirred for 1 hour. After reaction completion, phosphoric acid (85%wt, 45 μL) was added and the solution warmed up to room temperature. A batch distillation for solvent exchange was performed to change tetrahydrofuran with methanol (4 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (82:18).

**Comparative Example 20:** Preparation of a **82:18** mixture of (E) and (Z) acetyl voclosporin (Wittig reaction)

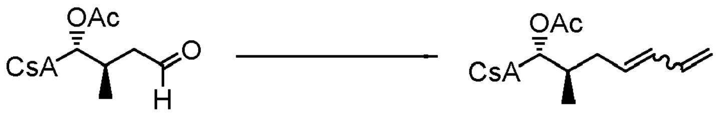

3 4

Cyclosporine-aldehyde **3** (1.0 g) was added to toluene (10 mL) and the mixture stirred until the material was completely dissolved. To the resulting solution allyl tributylphosphonium bromide (680 mg, 2.6 equivalent) was added. Potassium carbonate (270 mg, 2.4 equivalent) was added and the resulting mixture was stirred at 50 °C for 16 hour. After reaction completion, phosphoric acid (85%wt, 45 μL) was added and the solution warmed up to room temperature. A batch distillation for solvent exchange was performed to change toluene with methanol (4 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (82:18).

**Comparative Example 21:** Preparation of a **81:19** mixture of (E) and (Z) acetyl voclosporin (Wittig reaction)

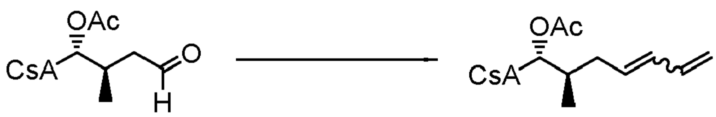

3 4

Cyclosporine-aldehyde **3** (1.0 g) was added to tetrahydrofuran (10 mL) and the mixture stirred until the material was completely dissolved. To the resulting solution allyl tributylphosphonium bromide (680 mg, 2.6 equivalent) was added. Sodium hydride 60% in mineral oil (80 mg, 2.4 equivalent) was added and the resulting mixture was stirred at 50 °C for 16 hour. After reaction completion, phosphoric acid (85%wt, 45 μL) was added and the solution warmed up to room temperature. A batch distillation for solvent exchange was performed to change tetrahydrofuran with methanol (4 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (82:18).

**Comparative Example 22:** Preparation of a **79:21** mixture of (E) and (Z) acetyl voclosporin (Wittig reaction)

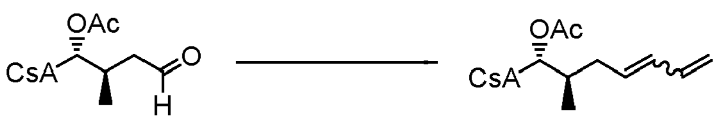

3 4

Allyl tributylphosphonium bromide (680 mg, 2.6 equivalent) was dissolved in toluene (10 mL) at -10°C. Hexilithium 2,3 M (0.85 mL, 2.4 equivalent) was added and the mixture was stirred for 30 min. Cyclosporine-aldehyde **3** (1.0 g) was added was added and the resulting mixture was stirred at -10 °C for 16 hour. After reaction completion, phosphoric acid (85%wt, 45 μL) was added and the solution warmed up to room temperature. A batch distillation for solvent exchange was performed to change toluene with methanol (4 mL). The resulting clear solution was stirred while water (12 mL) was added slowly at room temperature and then the resulting suspension was stirred for 1 hour, filtered and washed with methanol:water (3:6) (10 mL). The resulting wet cake was dried in a vacuum oven overnight (50°C) to give the product as mixture of isomers E:Z (79:21).

## Claims

1. Process for preparing a mixture of (E) and (Z)-Voclosporin of formula (I)

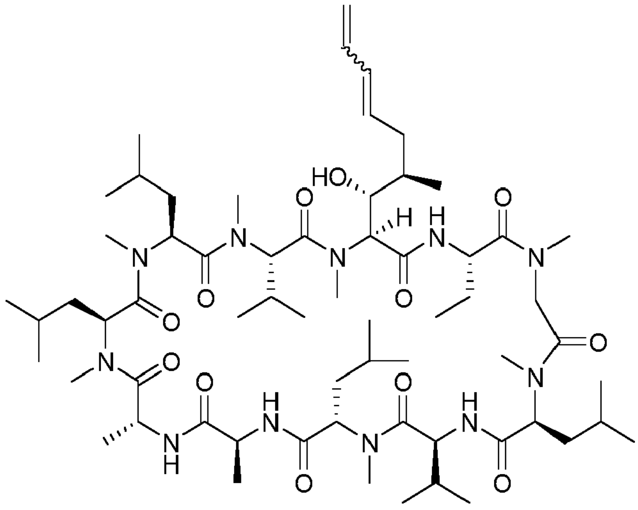

=

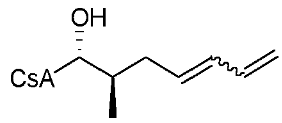

(I)

comprising:

a) reacting an aldehyde of formula (II),

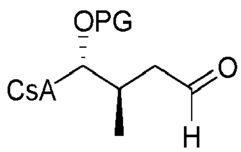

(II)

wherein PG is a hydroxyl protecting group, with a compound of formula (III)

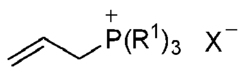

(III)

wherein

X is halogen, and
each $R^1$ is independently selected from $C_1$-$C_6$ alkyl and $C_6$-$C_{10}$ aryl,

in an amount of 0.2-1.0 molar equivalents with respect to the aldehyde of formula (II), in the presence of a base; and

b) reacting the product obtained from step a) with a compound of formula (IV)

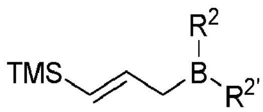

(IV)

wherein

$R^2$ and $R^{2'}$ are independently selected from the group consisting of $C_{1-6}$ alkyl, $C_{3-7}$ cycloalkyl, $OR^3$, or together they form a $C_{5-9}$ cycloalkyl group, or the -$B(R^2R^{2'})$ group is -$BF_3K$; wherein each $R^3$ is independently selected from the group consisting of H, $C_{1-6}$ alkyl, $C_{3-7}$ cycloalkyl, or together the two $R^3$ groups form a group selected from $C_{3-7}$ cycloalkyl, 5- to 7-membered heterocycloalkyl, 5- to 7-membered heteroaryl, $C_{6-10}$ aryl, and from $C_{2-5}$ alkylene optionally substituted by $C_{1-6}$ alkyl or -COO($C_{1-6}$ alkyl) wherein one of the carbon atoms in the $C_{2-5}$ alkylene group can be replaced by O, S or $NR^4$, wherein $R^4$ is selected from H and $C_{1-6}$ alkyl;
and an acid; and

c) deprotecting the hydroxyl protecting group.

**2.** Process according to claim 1, wherein the base in step b) is used in an amount of 0.18-0.95 molar equivalents with respect to the aldehyde of formula (II), preferably 0.18-0.76 molar equivalents.

**3.** Process according to any one of claims 1 or 2 for preparing a mixture of (E) and (Z)-Voclosporin in the ratio 87:13 to 97:3, preferably 90:10 to 96:4.

**4.** Process according to any one of claims 1 to 3, wherein PG is selected from:

- Si(R)(R')(R") where R, R' and R" are independently selected from $C_1$-$C_6$ alkyl, $C_3$-$C_7$ cycloalkyl, $C_6$-$C_{10}$ aryl, $C_1$-$C_6$ alkoxy and halogen,
- R or -$CH_2$-OR where R is selected from $C_1$-$C_6$ alkyl, $C_6$-$C_{10}$ aryl and ($C_6$-$C_{10}$)aryl($C_1$-$C_6$)alkyl,
- COR where R is selected from $C_1$-$C_6$ alkyl, $C_6$-$C_{10}$ aryl and ($C_6$-$C_{10}$)aryl($C_1$-$C_6$)alkyl, and
- COOR where R is selected from $C_1$-$C_6$ alkyl, $C_6$-$C_{10}$ aryl and ($C_6$-$C_{10}$)aryl($C_1$-$C_6$)alkyl;

preferably PG is a group -COR where R is selected from $C_1$-$C_6$ alkyl; more preferably PG is Ac.

**5.** Process according to any one of claims 1 to 4, wherein $R^1$ is selected from Ph and nBu.

**6.** Process according to any one of claims 1 to 5, wherein the compound of formula (III) is selected from allyl tributylphosphonium bromide, allyl triphenylphosphonium bromide and allyl triphenylphosphonium iodide.

**7.** Process according to any one of claims 1 to 6, wherein the base in step a) is selected from organolithium bases, organosodium bases, alkali metal hydrides, alkali metal hydroxides, alkali metal alkoxides, alkali metal carbonates and alkali metal phosphates and mixtures thereof, such as nBuLi, tBuLi, sBuLi, MeLi, HexLi, PhLi, HMDSLi, HMDSNa, LDA, NaH, LiOH, NaOH, KOH, LiOtBu, NaOtBu, KOtBu, LiOMe, LiOEt, NaOMe, NaOEt, KOMe, KOEt, $Na_2CO_3$, $K_2CO_3$, $Cs_2CO_3$, $Na_3PO_4$, $K_3PO_4$, and mixtures thereof.

**8.** Process according to any one of claims 1 to 7, wherein step a) is performed in the presence of an organic solvent, preferably selected from cyclic or acyclic ethers, hydrocarbon solvents, halogenated solvents, aromatic solvents, ketones, nitriles, amides, sulfoxides and mixtures thereof; more preferably cyclic ethers such as tetrahydrofuran or methyltetrahydrofuran.

**9.** Process according to any one of claims 1 to 8, wherein the compound of formula (III) is used in step a) in an amount of 0.2 to 0.9, preferably 0.2-0.8, molar equivalents with respect to the aldehyde of formula (II).

**10.** Process according to any one of claims 1 to 9, wherein in step a) the compound of formula (III) is tributylallylphosphonium bromide and the base is KOtBu and the reaction is performed in the presence of an organic solvent, preferably a cyclic or acyclic ether.

**11.** Process according to any one of claims 1 to 10, wherein the compound of formula (IV) is selected from:

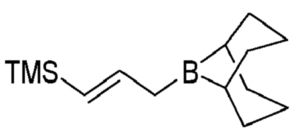,
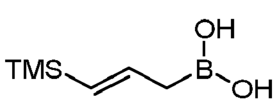,
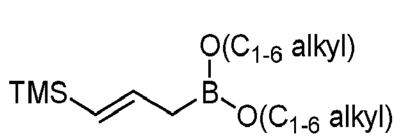,

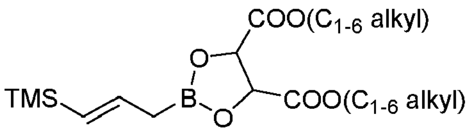, 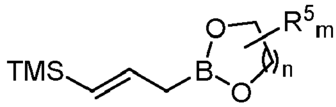, 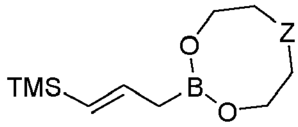

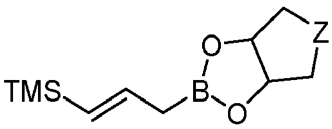, 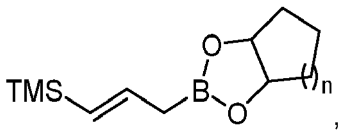, 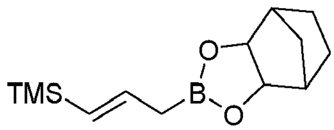

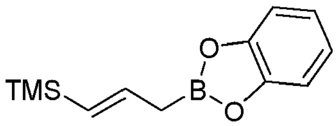 and 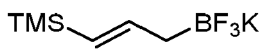

wherein

each $R^5$ is independently selected from $C_{1-6}$ alkyl,
n is an integer selected from 1 and 2,
m is an integer selected from 1, 2, 3 and 4, and
Z is selected from O, S and $NR^4$, wherein $R^4$ is selected from H and $C_{1-6}$ alkyl.

**12.** Process according to any one of claims 1 to 11, wherein step b) is performed in the presence of an organic solvent, preferably selected from cyclic or acyclic ethers, hydrocarbon solvents, halogenated solvents, aromatic solvents, esters, ketones, nitriles, amides, alcohols, sulfoxides and mixtures thereof; optionally in combination with water.

**13.** Process according to any one of claims 1 to 12, wherein the acid in step b) is an acid with a pKa lower than 4, preferably an acid selected from sulfuric acid, phosphoric acid, hydrochloric acid, formic acid, trifluoroacetic acid, methanesulfonic acid, tetrafluoroboric acid, perchloric acid, and mixtures thereof.

**14.** Process according to any one of claims 1 to 13, wherein steps a)-b) are performed in a one-pot manner.

**15.** Process according to any one of claims 1 to 14, wherein steps a) to b) are sequentially carried out in the presence of an organic solvent selected from cyclic or acyclic ethers, preferably THF, MeTHF or a mixture thereof.

Europäisches Patentamt
European Patent Office
Office européen des brevets

# EUROPEAN SEARCH REPORT

Application Number
EP 21 38 3182

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br><br><br>A | WO 03/033526 A2 (ISOTECHNIKA INC [CA]; HOFFMANN LA ROCHE [CH] ET AL.) 24 April 2003 (2003-04-24)<br>* pages 1, 10-12, 38-40; examples 19-29 * | 1-8, 10-15<br><br><br>9 | INV. C07K7/64 |
| A,D | WO 2006/014872 A2 (AMR TECHNOLOGY INC [US]; MOLINO BRUCE F [US] ET AL.) 9 February 2006 (2006-02-09)<br>* paragraphs [0006], [0014] - [0016]; claims 1-34 * | 1-15 | |

TECHNICAL FIELDS SEARCHED (IPC)
C07K

The present search report has been drawn up for all claims

| Place of search | Date of completion of the search | Examiner |
|---|---|---|
| The Hague | 16 May 2022 | Schmidt-Yodlee, H |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 21 38 3182

This annex lists the patent family members relating to the patent documents cited in the above-mentioned European search report. The members are as contained in the European Patent Office EDP file on
The European Patent Office is in no way liable for these particulars which are merely given for the purpose of information.

16-05-2022

| Patent document cited in search report | Publication date | Patent family member(s) | Publication date |
|---|---|---|---|
| WO 03033526 A2 | 24-04-2003 | AT 332917 T | 15-08-2006 |
| | | AT 425178 T | 15-03-2009 |
| | | AU 2010206069 A1 | 19-08-2010 |
| | | BR 0213658 A | 26-10-2004 |
| | | CA 2461740 A1 | 24-04-2003 |
| | | CN 1571795 A | 26-01-2005 |
| | | CO 5580836 A2 | 30-11-2005 |
| | | DE 60213115 T2 | 01-02-2007 |
| | | DK 1436321 T3 | 13-11-2006 |
| | | DK 1714977 T3 | 06-07-2009 |
| | | EP 1436321 A2 | 14-07-2004 |
| | | EP 1714977 A2 | 25-10-2006 |
| | | ES 2266564 T3 | 01-03-2007 |
| | | ES 2326040 T3 | 29-09-2009 |
| | | HK 1062568 A1 | 12-11-2004 |
| | | HR P20040355 A2 | 30-04-2005 |
| | | HR P20070058 A2 | 30-09-2008 |
| | | IL 160763 A | 18-11-2009 |
| | | IL 186140 A | 29-02-2012 |
| | | JP 4261355 B2 | 30-04-2009 |
| | | JP 4887469 B2 | 29-02-2012 |
| | | JP 5396571 B2 | 22-01-2014 |
| | | JP 2005516893 A | 09-06-2005 |
| | | JP 2009046515 A | 05-03-2009 |
| | | JP 2010280710 A | 16-12-2010 |
| | | KR 20050037417 A | 21-04-2005 |
| | | KR 20090130195 A | 18-12-2009 |
| | | MA 27293 A1 | 02-05-2005 |
| | | MX PA04003625 A | 02-12-2004 |
| | | NO 332459 B1 | 24-09-2012 |
| | | NZ 531944 A | 31-03-2006 |
| | | PL 210795 B1 | 30-03-2012 |
| | | PL 210841 B1 | 30-03-2012 |
| | | PT 1436321 E | 31-10-2006 |
| | | PT 1714977 E | 23-06-2009 |
| | | RU 2321593 C2 | 10-04-2008 |
| | | SI 1436321 T1 | 31-12-2006 |
| | | TN SN04068 A1 | 01-06-2006 |
| | | US RE48226 E | 29-09-2020 |
| | | US 2003139326 A1 | 24-07-2003 |
| | | US 2003212249 A1 | 13-11-2003 |
| | | US 2005192214 A1 | 01-09-2005 |
| | | US 2007087963 A1 | 19-04-2007 |
| | | US 2008171850 A1 | 17-07-2008 |
| | | US 2010062976 A1 | 11-03-2010 |
| | | US 2010311944 A1 | 09-12-2010 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 21 38 3182

This annex lists the patent family members relating to the patent documents cited in the above-mentioned European search report. The members are as contained in the European Patent Office EDP file on
The European Patent Office is in no way liable for these particulars which are merely given for the purpose of information.

16-05-2022

| Patent document cited in search report | Publication date | Patent family member(s) | Publication date |
|---|---|---|---|
| | | US 2013078280 A1 | 28-03-2013 |
| | | US 2014142277 A1 | 22-05-2014 |
| | | US 2014370082 A1 | 18-12-2014 |
| | | US 2016075741 A1 | 17-03-2016 |
| | | US 2017362277 A1 | 21-12-2017 |
| | | WO 03033526 A2 | 24-04-2003 |
| | | ZA 200402269 B | 25-05-2005 |
| WO 2006014872 A2 | 09-02-2006 | CA 2575280 A1 | 09-02-2006 |
| | | US 2008021197 A1 | 24-01-2008 |
| | | WO 2006014872 A2 | 09-02-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6605593 B
- US 6613739 B
- WO 1999018120 A
- WO 2003033526 A
- WO 2004089960 A
- WO 2006014872 A
- WO 2007112345 A
- WO 199918120 A
- WO 2003033527 A


### Non-patent literature cited in the description


- **ABEL et al.** ISATX247: A Novel Calcineurin Inhibitor. *J. Heart Lung Transplant,* 2001, vol. 20, 161
- **ASPESLET et al.** ISATX247: A Novel Calcineurin Inhibitor. *Transplantation Proceedings,* 2001, vol. 33, 1048-1051
- **GREEN TW et al.** Protective Groups in Organic Synthesis. 1999